# EUROPEAN PATENT SPECIFICATION

(11) **EP 3 189 135 B1**
(45) Date of publication and mention of the grant of the patent: **02.06.2021**
(21) Application number: 15838263.0
(22) Date of filing: 05.09.2015
(51) Int. Cl.: C12N 5/0793, C12N 5/0797, A61K 35/30, A61P 27/02

(54) **RETINAL GANGLION CELLS AND PROGENITORS THEREOF**
RETINALE GANGLIENZELLEN UND VORLÄUFER DAFÜR
CELLULES GANGLIONNAIRES DE LA RÉTINE ET LEURS PROGÉNITRICES

(30) Priority: 05.09.2014 US 201462046165 P
(43) Date of publication of application: 12.07.2017
(60) Divisional application: 21168050.9
(73) Proprietor: Astellas Institute for Regenerative Medicine, Westborough, MA 01581 (US)
(72) Inventor: WANG, Wei, Rochester, MN 55902 (US); LU, Shi-Jiang, Shrewsbury, MA 01545 (US); LANZA, Robert P., Clinton, MA 01510 (US)
(74) Representative: Boult Wade Tennant LLP
(86) International application number: PCT/US2015/048741
(87) International publication number: WO 2016/037159

(56) References cited:
- WO-A1-2011/028524
- US-A1- 2011 014 701
- YAO JING ET AL: "Math5 promotes retinal ganglion cell expression patterns in retinal progenitor cells.", MOLECULAR VISION 30 JUN 2007, vol. 13, 30 June 2007 (2007-06-30), pages 1066-1072, XP009504137, ISSN: 1090-0535
- HU YAMIN ET AL: "Anin vitrocomparison study: The effects of fetal bovine serum concentration on retinal progenitor cell multipotentiality", NEUROSCIENCE LETTERS, ELSEVIER, AMSTERDAM, NL, vol. 534, 12 November 2012 (2012-11-12), pages 90-95, XP028964259, ISSN: 0304-3940, DOI: 10.1016/J.NEULET.2012.11.006
- JOSEPH A BRZEZINSKI ET AL: "defines the ganglion cell competence state in a subpopulation of retinal progenitor cells exiting the cell cycle", DEVELOPMENTAL BIOLOGY, ELSEVIER, AMSTERDAM, NL, vol. 365, no. 2, 6 March 2012 (2012-03-06) , pages 395-413, XP028417326, ISSN: 0012-1606, DOI: 10.1016/J.YDBIO.2012.03.006 [retrieved on 2012-03-15]
- DATABASE BIOSIS [Online] BIOSCIENCES INFORMATION SERVICE, PHILADELPHIA, PA, US; May 2011 (2011-05), CHEN GUOCHUN ET AL: "Comparative Analysis Of Human Persistent Fetal Vascular Cells And Retinal Progenitor Cells As Candidates For Inner Retina Transplantation", XP009504142, Database accession no. PREV201200526948
- DATABASE BIOSIS [Online] BIOSCIENCES INFORMATION SERVICE, PHILADELPHIA, PA, US; October 2007 (2007-10), YAO JING ET AL: "Cultivation of retinal progenitor cells of different ages", XP009504138, Database accession no. PREV200800238558
- MU, XIUQIAN ET AL.: 'A gene network downstream of transcription factor Math 5 regulates retinal progenitor cell competence and ganglion cell fate' DEVELOPMENTAL BIOLOGY vol. 280, no. 2, 15 April 2005, pages 467 - 481, XP004908452
- XIANG, MENGQING ET AL.: 'Molecular control of retinal ganglion cell specification and differentiation' INTECH OPEN ACCESS PUBLISHER 2011, pages 65 - 84, XP055416300
- GILL, KATHERINE P. ET AL.: 'Methods of retinal ganglion cell differentiation from pluripotent stem cells' TRANSLATIONAL VISION SCIENCE & TECHNOLOGY vol. 3, no. 4, 01 July 2014, pages 1 - 12, XP055396469

## Description

### BACKGROUND OF THE INVENTION

Retinal diseases often result in blindness due to loss of post-mitotic neuronal cells. Among the retinal diseases are glaucoma, retinitis pigmentosa, rod or cone dystrophies, retinal degeneration, diabetic retinopathy, macular degeneration, Leber congenital amaurosis and Stargardt disease. Glaucoma, inter alia, involves injury or degeneration of retinal ganglion (RG) cells.

A potential replacement source of retinal ganglion (RG) cells is stem cells such as pluripotent stem cells. Early studies reported *in vitro* generation of retinal precursor cells from mouse embryonic stem cells (Ikeda et al. PNAS 102(32): 11331-11336, 2005), generation of retinal progenitor cells from postnatal day 1 mouse retinas (Klassen et al. Invest. Ophthal. Vis. Sci. 45(11):4167-4175, 2004), implantation of bone marrow mesenchymal stem cells in an RCS rat model of retinal degeneration (Inoue et al. Exp. Eye Res. 8(2):234-241, 2007), differentiation of induced pluripotent stem cells (iPS) from human fibroblasts to produce retinal progenitor cells (Lamba et al. PLoS ONE 5(1):e8763.doi:10.1371/journal.pone.0008763), and production of retinal progenitor cells, including amacrine cells, photoreceptors, bipolar cells and horizontal cells, from the HI human embryonic stem cell line (Lamba et al. Proc. Natl. Acad. Sci. 10(34): 12769-12774, 2006).

In these latter studies, the immature and mature retinal cells were generated as a mixed population of different cell types, and putative retinal ganglion cells represented a small percentage of the population. Moreover, the markers used to identify particular cell types, including neurofilament, Tuj 1 and HuC/D, are not specific to retinal ganglion (RG) cells and/or their precursors. One of these studies identified cells based on Brn3 expression, and found that few cells expressed the marker. Accordingly, none of these approaches produced a homogeneous, or near homogenous, population of retinal ganglion (RG) cells or of RG progenitor cells. None of these approaches produced sufficient numbers of RG cells or of RG progenitor cells, for example to be useful in *in vitro* methods, such as screening assays, or in *in vivo* methods.

Supplies of donor-derived tissue from which RG cells may be isolated (such as cadavers, fetal tissue, and live animals) are limited. Attempts to culture primary RG cells have resulted in cultures that lasted for at most about 2 weeks, after which the mature RG cells were non-viable. Isolation of primary RG progenitor cells has not been reported.

### SUMMARY OF THE INVENTION

Pluripotent stem cells can be propagated and expanded *in vitro* indefinitely, providing a potentially inexhaustible source of non-donor derived cells for human therapy. Differentiation of pluripotent stem cells into mature RG cells, including a homogeneous, or near homogeneous, population of mature RG cells and/or early and/or late RG progenitor cells, including a homogeneous, or near homogeneous, population of RG progenitor cells, may provide an abundant supply of non-donor derived cells for implantation and treatment of retinal diseases or *in vitro* uses such as screening assays. Highly purified populations of mature RG cells have not been previously obtained, and populations of RG progenitor cells of sufficient number or purity also have not been previously obtained. The invention overcomes at least these limitations of the prior art methods and compositions.

The invention provides, inter alia, compositions or preparations comprising RG progenitor cells and/or mature RG cells, as well as methods for preparing such cells and such preparations. Surprisingly, the RG progenitor cells and mature RG cells of the invention have been found to exhibit better integration and migration properties and longer survival times *in vivo* as compared to primary ganglion cells.

The present invention provides a method of producing a plurality of cells, comprising culturing eye field progenitor cells, as cell clusters and under low adherence or non-adherent conditions, in a cell media for a period of time sufficient for the cell clusters to form a plurality of cells,
wherein the eye field progenitor cells are capable of differentiating into retinal ganglion progenitor cells in the cell media, and wherein the cell media comprises forskolin, BDNF, and/or CNTF,
wherein the plurality of cells express:
one or more of Math5, Brn3a, Brn3b, Isl1, Neurofilament, and Thyl,
wherein the eye field progenitor cells are characterized as Pax6(+), Rx1(+), Oct4(-) and Nanog(-), and preferably are also characterized as Six3(+), Six6(+), Lhx2(+), Tbx3(+), Sox2(+), Otx2(+) and Nestin(+), as determined by immunostaining and/or flow cytometry.

The present invention also provides a composition of cells, comprising a plurality of cells at least 60% of which express
(i) one or more of Brn3a, Neurofilament, and Thyl, or
(ii) one or more of Math5, Brn3a, Brn3b, and Isl1,
as determined immunocytochemically,
and wherein the plurality of cells are formed as neurospheres.

In certain embodiments, the invention provides a preparation comprising a plurality of RG progenitor cells, and a medium suitable for maintaining the viability of the RG progenitor cells. Such medium may comprise glucose, insulin, factors that increase cAMP levels such as forskolin, and neurotrophic factors such as ciliary neurotrophic factor (CNTF) and brain derived neurotrophic factor (BDNF).

In certain embodiments, the invention provides a preparation of RG progenitor cells, comprising a plurality of cells containing at least 50% RG progenitor cells, and a medium suitable for maintaining the viability of the RG progenitor cells.

In certain embodiments, the invention provides a preparation of RG progenitor cells, comprising a plurality of RG progenitor cells substantially free of cells that are not RG progenitor cells such as pluripotent stem cells, eye field progenitor cells, photoreceptor progenitor cells, mature photoreceptors, and/or amacrine cells. In some embodiments, the preparation includes less than 10% of cells that are not RG progenitor cells, and even more preferably less than 5%, 2%, 1%, 0.1% or even less than 0.01% of those cells. In some embodiments, the preparation may be substantially free of RG cells (i.e., mature RG cells). Any of these preparations may further comprise a medium suitable for maintaining the viability of the RG progenitor cells.

In some embodiments, the preparation may comprise a mixture of mature RG cells and RG progenitors but may lack other cell types such as pluripotent stem cells, eye field progenitor cells, photoreceptor progenitor cells, mature photoreceptors, and/or amacrine cells.

In certain embodiments, the invention provides a pharmaceutical preparation of RG progenitor cells that is suitable for use in a mammalian patient, comprising a plurality of RG progenitor cells; and a pharmaceutically acceptable carrier for maintaining the viability of the RG progenitor cells for transplantation into a mammalian patient.

Also disclosed herein is a cryogenic cell preparation comprising at least 10⁹ RG progenitor cells, and a cryopreservative system compatible with the RG progenitor cells and able to maintain the viability of such cells after thawing.

In some embodiments of the preparations comprising RG progenitor cells, at least 70% of the cells in the preparation are immunocytochemically Math5(+), and even more preferably at least 80%, 90%, 95% or 98% of the cells in the preparation are immunocytochemically Math5(+). In some embodiments, cells in the preparation are also Brn3a(+). In some embodiments, cells in the preparation are also Brn3b(+). In some embodiments, cells in the preparation are also Isl1(+). In some embodiments, cells in the preparation are also Brn3a(+) and Brn3b(+). In some embodiments, cells in the preparation are also Brn3a(+), Brn3b(+) and Isl1(+).

In some preparations comprising RG progenitor cells, at least 70% of the cells in the preparation are immunocytochemically Math5(+) and Brn3a(+) (i.e., positive for Math5 and Brn3a), and even more preferably at least 80%, 90%, 95% or 98% of the cells in the preparation are immunocytochemically Math5(+) and Brn3a(+). In some embodiments, cells in the preparation are also Brn3b(+). In some embodiments, at least 50%, 60%, 70%, 80%, 90%, 95% or 98% of the cells in the preparation are immunocytochemically Math5(+), Brn3a(+) and Brn3b(+). In some embodiments, cells in the preparation are also Isl1(+). In some embodiments, at least 50%, 60%, 70%, 80%, 90%, 95% or 98% of the cells in the preparation are immunocytochemically Math5(+), Brn3a(+), Brn3b(+) and Isl(+). In some embodiments, less than 30%, 20%, 10%, 5%, 1% of cells or none of the cells express Thy1. The RG progenitor cells may also express Tuj 1.

In some preparations comprising RG progenitor cells, at least 50%, 60% or 70% of the cells in the preparation are immunocytochemically Brn3a(+) and/or Neurofilament(+), and even more preferably at least 80%, 90%, 95% or 98% of the cells in the preparation are immunocytochemically Brn3a(+) and/or Neurofilament(+). In some embodiments, at least 50%, 60% or 70% of the cells in the preparation are immunocytochemically Brn3a(+) and Neurofilament(+), and even more preferably at least 80%, 90%, 95% or 98% of the cells in the preparation are immunocytochemically Brn3a(+) and Neurofilament(+). In some embodiments, cells in the preparation are also Thyl(+). In some embodiments, at least 50%, 60% or 70% of the cells in the preparation are immunocytochemically Brn3a(+), Neurofilament(+) and Thyl(+). The RG progenitor cells may also express Tuj 1.

The RG progenitor cells are proliferative. In some embodiments, at least 70%, 80%, 90%, 95% or 98% of the cells in the preparations of RG progenitor cells are proliferative.

In certain embodiments, the RG progenitor cells are HLA-genotypically identical, and preferably are genomically identical.

In certain embodiments, the RG progenitor cells have a mean terminal restriction fragment length (TRF) that is longer than 7 kb, 7.5 kb, 8 kb, 8.5 kb, 9 kb, 9.5 kb, 10 kb, 10.5 kb, 11 kb, 11.5 kb or even 12 kb.

In certain embodiments, the RG progenitor cells are suitable for administration to a human patient.

In certain embodiments, the RG progenitor cells are suitable for administration to a non-human veterinarian patient.

In preferred embodiments of the above preparations, the RG progenitor cells are derived from mammalian pluripotent stem cells, especially human pluripotent stem cells, preferably selected from the group consisting of embryonic stem cells and induced pluripotent stem cells.

In certain embodiments, the RG progenitor cells are differentiated from a common pluripotent stem cell source.

The RG progenitor cells can be transplanted into the vitreous or subretinal space of intraocular hypertension glaucoma mice or rat model systems, or of optical nerve (ON) injury crush mice model systems, and will migrate to the ganglion cell layer and will improve pattern ERG (electroretinography) responses and visual acuity in any of these model systems. In certain embodiments, the RG cells and RG progenitor cells may regenerate the optical nerve.

In certain embodiments, the RG progenitor cells and RG cells secrete one or more neuroprotective factors, and thereby provide neuroprotection. Such neuroprotection may be measured using animal models of optic nerve injury or glaucoma.

In certain embodiments, the medium suitable for maintaining the viability of the RG progenitor cells is selected from the group consisting of a culture medium, a cryopreservative, and a biocompatible injection medium suitable for injection in a human patient.

In certain embodiments, the RG progenitor cell preparation is pyrogen and mycogen free.

Another aspect of the present invention provides a pharmaceutical preparation of RG cells that is suitable for use in a mammalian patient, comprising pluripotent stem cell derived RG cells, wherein cells in the preparation are immunocytochemically positive for Neurofilament and/or Brn3a, and thus may be in some instance Neurofilament(+) Brn3a(+). The RG cells may be Tuj1(+), and they may also optionally be Thyl(+). The pharmaceutical preparations may further comprise a pharmaceutically acceptable carrier for maintaining the viability of the RG cells for transplantation into a mammalian patient.

Another aspect of the present invention provides a pharmaceutical preparation of RG cells that is suitable for use in a mammalian patient, comprising pluripotent stem cell derived RG cells, wherein cells in the preparation are immunocytochemically Neurofilament(+) Brn3a(+),and Tuj1(+), and optionally Thy1(+), and a pharmaceutically acceptable carrier for maintaining the viability of the RG cells for transplantation into a mammalian patient.

Another aspect of the present invention provides a pharmaceutical preparation of RG cells that is suitable for use in a mammalian patient, comprising pluripotent stem cell derived RG cells, wherein greater than 70%, 80%, 90%, 95% or even 98% of the cells are immunocytochemically Neurofilament(+) and Brn3a(+), and a pharmaceutically acceptable carrier for maintaining the viability of the RG cells for transplantation into a mammalian patient. In some instances, greater than 50%, 60% or 70% of the cells in the preparation are immunocytochemically Thyl(+). In some embodiments, at least 70%, 80%, 90%, 95% or even 98% of the cells are immunocytochemically Tuj1(+).

In some embodiments, cells in the pharmaceutical preparation of RG cells are mitotically inactive (or post-mitotic), meaning that they are non-proliferative. This can be determined using proliferation assays known in the art such as tritiated thymidine uptake assays, etc. In some embodiments, greater than 50%, 60%, 70%, 80%, 90%, 95%, or 98% of the cells in the preparation are mitotically inactive.

In some embodiments, cells in the pharmaceutical preparation of RG cells comprise a greater number of dendrites and/or more complex dendrites than an RG progenitor class. In some embodiments, greater than 50%, 60%, 70%, 80%, 90%, 95%, or 98% of the cells in the preparation comprise a greater number of dendrites and/or more complex dendrites (as compared to the progenitor classes). Thus, mature RG cells exhibit an increased number of primary dendrites and/or increased dendritic branching at secondary, tertiary and higher levels, and/or increased total dendritic length compared to RG progenitor cells. For example, RG cells may have 2-5 fold more dendrites on a per cell basis than RG progenitor cells (e.g., RG cells may have 4-5 dendrites per cell and RG progenitor cells may have 1-2 dendrites per cell).

Still another aspect of the present invention provides a pharmaceutical preparation comprising RG progenitor cells and/or RG cells together with retinal pigment epithelial cells, photoreceptor progenitor cells, and/or photoreceptor cells; and a pharmaceutically acceptable carrier for maintaining the viability of the RG progenitor cells and/or the RG cells for transplantation into a mammalian patient. The preparation may also include bipolar cells and/or amacrine cells. The preparation of cells can be provided as cell suspensions (either admixed together, or in the form of a kit with separate doses of cells to be delivered conjointly), as three-dimensional structures, including for example as a sheet or monolayer or multi-layer cell graft (optionally disposed on a biocompatible matrix or solid support). In the case of the multi-layer cell graft, the RPE cells can be provided as a monolayer, preferably a polarized monolayer. Thus, in some embodiments, the RG cells and/or RG progenitor cells are used together with other neuroretinal sensory cells including RPE cells, photoreceptor cells or photoreceptor progenitor cells.

Yet another aspect of the invention provides a composition for use in a method of treatment of a mammalian patient in need thereof. Such methods include methods for treating diseases and disorders caused by loss or dysfunction of retinal ganglion cells in a patient, comprising administering such pharmaceutical preparations as described herein, such as preparations of RG progenitor cells or RG cells, or both. The preparations can be injected locally, such as into the sub-retinal space of the patient's eye, into the vitreous of the patient's eye, or delivered systemically or into other body cavities where the cells can persist.

The diseases or disorders caused by loss of retinal ganglion cells include glaucoma, optic nerve injury, ischemic optic neuropathy, optic neuritis, diabetic retinopathy, inherited ganglion degeneration, and inherited retinal dystrophy.

Thus also disclosed herein is the use of any of the RG progenitor cell populations described herein and/or made using the methods described herein in the manufacture of a medicament for the treatment of a disease or disorder caused by loss or dysfunction of retinal ganglion cells in a subject. Also provided are compositions comprising the RG progenitor cell populations described herein for use in a method of treating a mammalian patient in need thereof, including treating a disease or disorder caused by loss or dysfunction of retinal ganglion cells in a subject.

Similarly also disclosed herein is the use of any of the RG cell populations described herein and/or made using the methods described herein in the manufacture of a medicament for the treatment of a disease or disorder caused by loss or dysfunction of retinal ganglion cells in a subject. Also provided are compositions comprising the RG cell populations described herein for use in a method of treating a mammalian patient in need thereof, including treating a disease or disorder caused by loss or dysfunction of retinal ganglion cells in a subject.

In certain embodiments, the invention provides a method of producing RG cells, comprising the steps of
(a) culturing eye field progenitor cells, preferably as cell clusters and preferably under low adherence or non-adherent conditions, in a ganglion cell medium for a period of time sufficient for the cell clusters to form individual cell spheres comprising RG progenitor cells; and
(b) culturing the cell spheres in a retinal ganglion cell differentiation medium under adherent conditions, preferably on a matrix (such as a biomaterial scaffold) until a majority of cells in the culture are RG cells characterized as Brn3a(+) and/or Neurofilament(+) (and preferably Brn3a(+) and Neurofilament(+)) and Tuj1(+), and optionally also Thyl(+).

In certain embodiments, the invention provides a method, comprising culturing eye field progenitor cells, preferably as cell clusters and preferably under low adherence or non-adherent conditions, in a ganglion cell medium for a period of time sufficient for the cell clusters to form RG progenitor cells. The RG progenitor cells may be optionally cultured in a retinal ganglion cell differentiation medium under adherent conditions, preferably on a matrix (such as a biomaterial scaffold) until a majority of cells in the culture are RG cells characterized as Brn3a(+), Neurofilament(+) and Tuj 1(+), and optionally Thyl(+).

In accordance with the claims, the eye field progenitor cells are characterized, such as immunocytochemically characterized, as Pax6(+) and Rx1(+) and Oct4(-) and Nanog(-), and even more preferably are also characterized as Six3(+), Six6(+), Lhx2(+), Tbx3(+), Sox2(+), Otx2(+) and Nestin(+), such as may be determined by immunostaining and/or flow cytometry.

In some embodiments, the RG progenitor cells are characterized as Math5(+), or as Math5(+) and Brn3a(+), or as Math5(+), Brn3a(+) and Brn3b(+), or as Math5(+), Brn3a(+), Brn3b(+) and Isl1(+), or as Brn3a(+) and Neurofilament(+), or as Brn3a(+), Neurofilament(+) and Thy1(+), such as may be determined by immunostaining and/or flow cytometry or other standard assay used to characterize marker expression in cells.

In certain embodiments, the adherent conditions include a culture system having a surface to which the cells can adhere that includes an adherent material, which may comprise, merely to illustrate, one or more of a polyester, a polypropylene, a polyalkylene, a polyfluorochloroethylene, a polyvinyl chloride, a polyvinyl fluoride resin, a polystyrene, a polysulfone, a polyurethane, a polyethyene terephtalate, a cellulose, a glass fiber, a ceramic particle, a biomaterial scaffold, a poly-L-lactic acid, a dextran, an inert metal fiber, silica, natron glass, borosilicate glass, chitosan, or a vegetable sponge. In some embodiments, the adherent material is electrostatically charged. In certain embodiments, the biomaterial scaffold is extracellular matrix, such as collagen (such as collagen type IV or type I), 804G-derived matrix, fibronectin, vitronectin, chondronectin, laminin or Matrigel™. In other embodiments, the biomaterial is gelatin, alginate, polyglycolide, fibrin, or self-assembling peptides.

In certain embodiments, the RG progenitor cells, and as a consequence the RG cells, are derived from pluripotent stem cells, such as embryonic stem cells or induced pluripotent stem cells.

In preferred embodiments, the resulting preparation of RG progenitor cells, are provided substantially free of pluripotent stem cells, i.e., less than 10% of the cells in the preparation are pluripotent stem cells, and even more preferably less than 5%, 2%, 1%, 0.1% or even less than 0.01% of the cells in the preparation are pluripotent stem cells.

In preferred embodiments, the resulting preparation of RG progenitor cells are provided substantially free of eye field progenitor cells, i.e., less than 10%, and even more preferably less than 5%, 2%, 1%, 0.1% or even less than 0.01% of the cells in the preparation are eye field progenitor cells.

In preferred embodiments, the cellular component of the resulting preparation of RG progenitor cells is at least 50% pure with respect to other cell types (i.e., at least 50% of the cells in the preparation are RG progenitor cells), and preferably at least 75%, at least 85%, at least 95%, at least 99% or about 100% of the cells in the preparation are RG progenitors.

In certain embodiments, the method includes the further step of cryopreserving the RG progenitor cells or the RG cells. The cells are preferably frozen in a cryopreservative which is compatible with the thawing of the frozen cells (and the thawed cells themselves) and, after optionally washing the cells to remove the cryopreservative, the RG progenitor cells or the RG cells retain at least 25% cell viability (such as based on culture efficiency), and more preferably at least 50%, 60%, 70%, 80% or even at least 90% cell viability.

The RG progenitor cells or the RG cells may be cryopreserved. In some embodiments, the RG progenitor cells are cryopreserved as spheres.

In certain embodiments, the ganglion cell medium may comprise a basal medium such as Neurobasal™ Medium (Life Technologies) (or other medium comprising similar constituents), D-glucose, antibiotics such as penicillin and streptomycin, GlutaMAX™, N2 supplement (Invitrogen), B27 supplement (also from Invitrogen), forskolin, BDNF and CNTF. B27 supplement contains, inter alia, SOD, catalase and other anti-oxidants (GSH), and unique fatty acids, such as linoleic acid, linolenic acid, and lipoic acids. N2 supplement can be replaced with, for example, the following cocktail: transferrin (10 g/L), insulin (500 mg/L), progesterone (0.63 mg/L), putrescine (1611 mg/L) and selenite (0.52 mg/L).

In certain embodiments of the foregoing aspects and embodiments, the RG progenitor cells are differentiated from a pluripotent stem cell source, such as a pluripotent stem cell that expresses Oct4, alkaline phosphatase, Sox2, SSEA-3, SSEA-4, TRA-1-60, and TRA-1-80 (such as an embryonic stem (ES) cell line or induced pluripotency stem (iPS) cell line), and even more preferably from a common pluripotent stem cell source.

In certain embodiments of the foregoing aspects and embodiments, the RG progenitor cells have a mean terminal restriction fragment length (TRF) that is longer than 7 kb, 7.5 kb, 8 kb, 8.5 kb, 9 kb, 9.5 kb, 10 kb, 10.5 kb, 11 kb, 11.5 kb or even 12 kb.

In certain embodiments of the foregoing aspects and embodiments, a preparation is suitable for administration to a human patient, and more preferably pyrogen-free and/or free of non-human animal products.

In certain embodiments of the foregoing aspects and embodiments, a preparation is suitable for administration to a non-human veterinarian mammal, such as a dog, cat or horse.

In one aspect, the disclosure provides a method of producing RG progenitor cells, comprising culturing pluripotent stem cells, sequentially, in (i) a retinal induction (RI) medium, (ii) neural differentiation (ND) medium, and (iii) ganglion cell (GC) medium.

The pluripotent stem cells may be human. The pluripotent stem cells may be human ES cells or human iPS cells, in some embodiments. The pluripotent stem cells may be cultured, in an undifferentiated state, under feeder-free and/or xeno-free conditions, optionally in the presence of a matrix. The pluripotent stem cells may be cultured, in an undifferentiated state, on a substrate such as a substrate comprising Matrigel™ and optionally in mTESR1 medium.

Differentiation commences by culturing the pluripotent stem cells in retinal induction medium. The retinal induction medium may comprise a basal medium such as DMEM/F12, DMEM/high glucose, or DMEM/knock-out, or a medium comprising the components of DMEM/F12, or a medium comprising the components of DMEM/high glucose, or a medium comprising the components of DMEM/knock-out.

The retinal induction medium may comprise D-glucose.

The neural differentiation medium may comprise D-glucose. D-glucose may be present in a concentration between 0-10 mg/ml, 2.5-7.5 mg/ml, 3-6 mg/ml, 4-5 mg/ml, or about 4.5 mg/ml.

The retinal induction medium may comprise one or more antibiotics. The antibiotics may include either or both penicillin and streptomycin, optionally in concentrations of about 0-100 units/ml or about 100 units/ml of penicillin and optionally about 0-100 µg/ml or about 100 µg/ml of streptomycin.

The retinal induction medium may comprise one or more serum supplements. The retinal induction medium may comprise N2 supplement. The N2 supplement may be present in a concentration of about 0.1 to 5% or about 0.1 to 2% or about 1% (volume/volume, or v/v).

The retinal induction medium may comprise B-27 supplement. The B-27 supplement may be present in a concentration of about 0.05-2.0% or about 0.2% (v/v).

The retinal induction medium may comprise non-essential amino acids or minimal essential medium (MEM) non-essential amino acids. The non-essential amino acids or MEM non-essential amino acids may be present at a IX concentration from stock, wherein stock is typically considered 100X. The final concentration of glycine (at the IX concentration of stock) is about 0.1 mM, and thus the stock is 10 mM glycine. The stock typically also includes L-alanine, L-asparagine, L-aspartic acid, L-glutamic acid, L-proline, and L-serine.

The retinal induction medium may comprise insulin. The insulin may be human. The insulin may be present in a concentration of about 5-50 µg/ml or about 20 µg/ml.

The retinal induction medium may comprise a BMP signaling inhibitor. The BMP signaling inhibitor may be selected from the group consisting of Noggin polypeptide, dorsomorphin, LDN-193189, and any combination thereof.

The retinal induction medium may comprise Noggin polypeptide. Noggin polypeptide may be present at a concentration of between about 5-100 ng/ml or about 10-100 ng/ml or about 10-500 ng/ml or about 50 ng/ml. In some embodiments, Noggin polypeptide is present at about 10-500 ng/ml.

The retinal induction medium may be replaced, in whole or in part, with fresh retinal induction medium daily. In some instances, the cells are grown at a density in the range of 2 x 10⁴/cm² to 6 x 10⁴/cm².

Culturing in the presence of retinal induction medium may occur for about 4-6 days, or about 4 days, about 5 days, or about 6 days.

The neural differentiation medium may comprise a basal medium such as Neurobasal™ Medium (Life Technologies), or a basal medium comprising components of Neurobasal™ Medium.

The neural differentiation medium may comprise D-glucose. D-glucose may be present in a concentration between 0-10 mg/ml, 2.5-7.5 mg/ml, 3-6 mg/ml, 4-5 mg/ml, or about 4.5 mg/ml.

The neural differentiation medium may comprise one or more antibiotics. The antibiotics may include either or both penicillin and streptomycin, optionally in concentrations of between 0-100 units/ml or about 100 units/ml of penicillin and optionally between 0-100 µg/ml or about 100 µg/ml of streptomycin.

The neural differentiation medium may comprise one or more serum supplements. The neural differentiation medium may comprise N2 supplement. The N2 supplement may be present in a concentration of about 0.1 to 5% or 0.1 to 2% or about 1% (v/v).

The neural differentiation medium may comprise B-27 supplement (Life Technologies). The B-27 supplement may be present in a concentration of about 0.05 to 5.0% , about 0.5 to 2.0% or about 2% (v/v).

The neural differentiation medium may comprise non-essential amino acids or MEM non-essential amino acids or glutamine or GlutaMAX™. The non-essential amino acids or MEM non-essential amino acids may be present at about a IX concentration of stock, wherein stock is about 200 mM (and considered 100X). GlutaMAX™ may be present at 2 mM or a IX dilution of stock (using a 100X stock solution that is 200 mM).

The neural differentiation medium may comprise a BMP signaling inhibitor. The BMP signaling inhibitor may be selected from the group consisting of Noggin polypeptide, dorsomorphin, LDN-193189, and any combination thereof.

The neural differentiation culture medium may comprise Noggin polypeptide. Noggin polypeptide may be present at a concentration of between about 10 to 1000 ng/ml, or about 10 to 500 ng/ml, or 10 to 100 ng/ml, or about 50 ng/ml.

Culturing in the presence of neural differentiation medium may occur for about 8-12 days, or about 9-10 days, or about 9 days. The neural differentiation medium may be changed, in whole or in part, daily, or every 2 days, or every three days.

The ganglion cell medium may comprise a basal medium such as Neurobasal™ Medium (Life Technologies) or a medium comprising the components of Neurobasal™ Medium.

The ganglion cell medium may comprise D-glucose. The neural differentiation medium may comprise D-glucose. D-glucose may be present in a concentration between 0-10 mg/ml, 2.5-7.5 mg/ml, 3-6 mg/ml, 4-5 mg/ml, or about 4.5 mg/ml.

The ganglion cell medium may comprise one or more antibiotics. The antibiotics may include either or both penicillin and streptomycin, optionally in concentrations of between 0-100 units/ml or about 100 units/ml of penicillin and optionally between 0-100 µg/ml or about 100 µg/ml of streptomycin.

The ganglion cell medium may comprise one or more serum supplements. The ganglion cell medium may comprise N2 supplement. The N2 supplement may be present in a concentration of about 0.1 to 5% or 0.1 to 2% or about 1% (v/v).

The ganglion cell medium may comprise B-27 supplement (formula number 080085-SA) (Life Technologies). The B-27 supplement (formula number 080085-SA) may be present in a concentration of about 0.05 to 5.0% , about 1.5 to 2.5% or about 2% (v/v).

The ganglion cell medium may comprise GlutaMAX™. GlutaMAX™ may be present at a IX dilution of stock (using a 100X stock solution that is 200 mM).

The ganglion cell medium may comprise one, two or all of forskolin, BDNF and CNTF. Forskolin may be present in a concentration of 1-20 µM or 1-10 µM or about 5 µM. BDNF may be present at a concentration of 1-200 ng/ml or 5-50 ng/ml or 5-25 ng/ml or about 10 ng/ml. CNTF may be present at a concentration of 1-200 ng/ml or 5-50 ng/ml or 5-25 ng/ml or about 10 ng/ml. BDNF may be human BDNF. CTNF may be human CTNF.

Culturing in the presence of ganglion cell medium may occur for about 40-50 days, or about 45 days. The ganglion cell medium may be changed, in whole or in part, daily, or every 2 days, or every three days, or less frequently.

The RG progenitor cells produced by the method may comprise at least 50%, at least 75%, at least 85%, at least 95%, at least 99% or about 100% of the cells in the culture, and optionally such culture may be propagated for 1 week, 2 weeks, or more than 2 weeks, including 3 weeks, 4 weeks, or longer.

The RG progenitor cells may express one or more of the markers: Math5, Brn3a, Brn3b, Isl1, Neurofilament, and Thyl, and thus may be characterized as Math5(+), Brn3a(+), Brn3b(+), Isl1(+), Neurofilament(+), and/or Thyl(+). The RG progenitor cells may express one or more of the markers: Math5, Brn3a, Brn3b, and Isl1, and thus may be characterized as Math5(+), Brn3a(+), Brn3b(+), and/or Isl1(+). The RG progenitor cells may express one or more of the markers: Brn3a, Neurofilament, and Thyl, and thus may be characterized as Brn3a(+), Neurofilament(+), and/or Thyl(+). The RG progenitors may not express Pax6 and/or Rx1, and thus may be characterized as Pax6(-) and/or Rx1(-). Certain RG progenitor cells may not express Math5 and/or Brn3b, and thus may be characterized as Math5(-) and/or Brn3b(-).

The RG progenitor cells may be characterized as Math5(+), or Math5(+), Brn3a(+), or Math5(+), Brn3a(+), Brn3b(+), or Math5(+), Brn3a(+), Brn3b(+), Isl1(+), or Brn3a(+), or Neurofilament(+), or Brn3a(+), Neurofilament(+), or Brn3a(+), Neurofilament(+), Thyl(+). The RG progenitors may not express Pax6 and/or Rx1, and thus may be characterized as Pax6(-) and/or Rx1(-). The RG progenitor cells may be Tuj1(+). The RG progenitor cells may be proliferative.

The RG progenitor cells may be cryopreserved. The RG progenitor cells may be human.

The method may further comprise a subsequent culturing step in the presence of retinal ganglion cell (RGC) differentiation medium. This may occur without an intermediate cryopreservation step or it may occur after cryopreservation and thawing of the RG progenitor cells.

The RGC differentiation medium may comprise a basal medium such as Neurobasal™ Medium (Life Technologies) or a medium comprising the components of Neurobasal™ Medium.

The RGC differentiation medium may comprise D-glucose. The neural differentiation medium may comprise D-glucose. D-glucose may be present in a concentration between 0-10 mg/ml, 2.5-7.5 mg/ml, 3-6 mg/ml, 4-5 mg/ml, or about 4.5 mg/ml.

The RGC differentiation medium may comprise one or more antibiotics. The antibiotics may include either or both penicillin and streptomycin, optionally in concentrations of between 0-100 units/ml or about 100 units/ml of penicillin and optionally between 0-100 µg/ml or about 100 µg/ml of streptomycin.

The RGC differentiation medium may comprise one or more serum supplements. The RGC differentiation medium may comprise B-27 supplement (formula 080085-SA). The B-27 supplement may be present in a concentration of about 0.05 to 5.0%, about 1.5 to 2.5% or about 2% (v/v).

The RGC differentiation medium may comprise GlutaMAX™. GlutaMAX™ may be present at a IX dilution of stock.

The RGC differentiation medium may comprise retinoic acid. Retinoic acid may be present at a concentration of about 0.5-20 µM, about 0.5-10 µM, about 1-5 µM, or about 2 µM.

The RGC differentiation medium may comprise one or more including all of forskolin, BDNF, CNTF, cAMP, and DAPT. The RGC differentiation medium may comprise one or both of BDNF and CNTF. The RGC differentiation medium may comprise one or more including all of forskolin, cAMP, and DAPT or other Notch pathway inhibitor or Notch inhibitor (such as Notch blocking antibody or antibody fragment, Notch negative regulatory region antibody or antibody fragment, alpha-secretase inhibitor, gamma-secretase inhibitor, stapled peptide, small molecule blockers and siRNA, shRNA and miRNA). Forskolin may be present in a concentration of 1-20 µM or 1-10 µM or about 5 µM. BDNF may be present at a concentration of 1-200 ng/ml or 5-50 ng/ml or 5-25 ng/ml or about 10 ng/ml. CNTF may be present at a concentration of 1-200 ng/ml or 5-50 ng/ml or 5-25 ng/ml or about 10 ng/ml. BDNF may be human BDNF. CTNF may be human CTNF. cAMP may be present at a concentration of 1-500 ng/ml or 10-250 ng/ml or 5-150 ng/ml or about 100 ng/ml. DAPT may be present at a concentration of 1-100 µM or 1-50 µM or 1-20 µM, or about 10 µM.

The RGC differentiation medium may comprise insulin. The insulin may be human. The insulin may be present in a concentration of about 5-50 µg/ml or about 20 µg/ml.

Culturing in the presence of RGC differentiation medium may occur for about 1-4 weeks or about 2-4 weeks or about 2-3 weeks or about 2 weeks. The RGC differentiation medium may be changed, in whole or in part, daily, or every 2 days, or every three days, or less frequently.

The RG cells produced by the method may comprise at least 50%, at least 75%, at least 85%, at least 95%, at least 99% or about 100% of the cells in the culture, and optionally such culture may be propagated for 1 week, 2 weeks, or more than 2 weeks, including 3 weeks, 4 weeks, or longer.

The RG cells may express one or more of the markers: Brn3a, Neurofilament, Tuj 1 and Thyl, and thus may be characterized as Brn3a(+), Neurofilament(+), Tuj1(+), and/or Thyl(+). The RG cells may be characterized as Brn3a(+), Neurofilament(+), or as Brn3a(+), Neurofilament(+), Thy1(+), or as Brn3a(+), Neurofilament(+), Thy1(+), or as Brn3a(+), Neurofilament(+), Tuj1(+), or as Brn3a(+), Neurofilament(+), Tuj1(+), Thyl(+). The RG cells may be post-mitotic and may comprise a higher number, longer and/or more complex dendrites than RG progenitor cells.

The RG cells may be cryopreserved. The RG cells may be human.

In another aspect, the disclosure provides a method of producing RG progenitor cells, comprising culturing eye field progenitor cells in a ganglion cell medium.

The eye field progenitor cells may be provided as a population of cells, at least 50%, at least 75%, at least 85%, at least 95%, at least 99% or about 100% of which are eye field progenitor cells. The eye field progenitor cells may be Pax6(+) and Rx1(+) and optionally also Six3(+), Six6(+), Lhx2(+), and/or Tbx3(+). The eye field progenitor cells may also be Sox2(+). The eye field progenitor cells may be Nestin(+). The eye field progenitor cells may also be Otx2(+).

The eye field progenitors may be obtained by *in vitro* differentiation of pluripotent stem cells. The pluripotent stem cells may be ES cells or iPS cells, in some embodiments. The pluripotent stem cells may be cultured under feeder-free and/or xeno-free conditions.

The culture of eye field progenitor cells may occur for about 5-45 days, or about 5-20 days, or about 35-45 days. The ganglion cell medium and culture parameters in the ganglion cell medium may be as described above.

The RG progenitor cells produced by the method may comprise at least 50%, at least 75%, at least 85%, at least 95%, at least 99% or about 100% of the cells in the culture, and optionally such culture (comprising such % of RG progenitor cells) may be propagated for 1 week, 2 weeks, or more than 2 weeks, including 3 weeks, 4 weeks, or longer.

The RG progenitor cell phenotype is as described above. Thus, as an example, the RG progenitor cells may express one or more of the markers: Math5, Brn3a, Brn3b, Isl1, Neurofilament, and Thyl, and thus may be characterized as Math5(+), Brn3a(+), Brn3b(+), Isl1(+), Neurofilament(+), and/or Thyl(+). The RG progenitor cells may express one or more of the markers: Math5, Brn3a, Brn3b, and Isl1, and thus may be characterized as Math5(+), Brn3a(+), Brn3b(+), and/or Isl1(+). The RG progenitor cells may express one or more of the markers: Brn3a, Neurofilament, and Thyl, and thus may be characterized as Brn3a(+), Neurofilament(+), and/or Thyl(+). The RG progenitors may not express Pax6 or Rx1, and thus may be characterized as Pax6(-) and/or Rx1(-). The RG progenitor cells may be proliferative. Other phenotypes are described above.

The RG progenitor cells may be cryopreserved. The RG progenitor cells may be human.

In another aspect, the disclosure provides a method of producing RG cells, comprising culturing eye field progenitor cells sequentially in a ganglion cell medium and a retinal ganglion cell (RGC) differentiation medium.

The eye field progenitor cells may be provided as a population of cells, at least 50%, at least 75%, at least 85%, at least 95%, at least 99% or about 100% of which are eye field progenitor cells. The eye field progenitor cells may be Pax6(+) and Rx1(+) and optionally also Six3(+), Six6(+), Lhx2(+), and/or Tbx3(+). The eye field progenitor cells may also be Sox2(+). The eye field progenitor cells may be Nestin(+). The eye field progenitor cells may also be Otx2(+).

The eye field progenitors may be obtained by *in vitro* differentiation of pluripotent stem cells. The pluripotent stem cells may be ES cells or iPS cells, in some embodiments. The pluripotent stem cells may be cultured under feeder-free and/or xeno-free conditions.

The ganglion cell medium and culture parameters in the ganglion cell medium may be as described above. The RGC differentiation medium and culture parameters in the RGC differentiation medium may be as described above.

The RG cells produced by the method may comprise at least 50%, at least 75%, at least 85%, at least 95%, at least 99% or about 100% of the cells in the culture, and optionally such culture (comprising such % of RG cells) may be propagated for 1 week, 2 weeks, or more than 2 weeks, including 3 weeks, 4 weeks, or longer.

The phenotype of the RG cells is as described above. Thus, as an example, the RG cells may express one or more of the markers: Brn3a, Neurofilament, Tuj1, and Thyl, and thus may be characterized as Brn3a(+), Neurofilament(+), Tuj1(+), and/or Thyl(+). The RG cells may be post-mitotic. The RG cells may have more complex dendrites, longer dendrites, and/or more dendrites than RG progenitor cells. Other phenotypes are described above.

The RG cells may be cryopreserved. The RG cells may be human.

In another aspect, the disclosure provides a method of producing RG cells, comprising culturing RG progenitor cells in a retinal ganglion cell (RGC) differentiation medium.

The RG progenitor cells may be provided as a population of cells, at least 50%, at least 75%, at least 85%, at least 95%, at least 99% or about 100% of which are RG progenitor cells. The RG progenitor cells may express one or more of the markers: Math5, Brn3a, Brn3b, Isl1, Neurofilament, and Thyl, and thus may be characterized as Math5(+), Brn3a(+), Brn3b(+), Isl1(+), Neurofilament(+), and/or Thyl(+). The RG progenitor cells may express one or more of the markers: Math5, Brn3a, Brn3b, and Isl1, and thus may be characterized as Math5(+), Brn3a(+), Brn3b(+), and/or Isl1(+). The RG progenitor cells may express one or more of the markers: Brn3a, Neurofilament, and Thyl, and thus may be characterized as Brn3a(+), Neurofilament(+), and/or Thyl(+). The RG progenitors may not express Pax6 or Rx1 and thus may be characterized as Pax6(-) and/or Rx1(-). The RG progenitors may be obtained by *in vitro* differentiation of pluripotent stem cells. The pluripotent stem cells may be ES cells or iPS cells, in some embodiments. The pluripotent stem cells may be cultured under feeder-free and/or xeno-free conditions.

The RGC differentiation medium and culture parameters in the RGC differentiation medium may be as described above.

The RG cells produced by the method may comprise at least 50%, at least 75%, at least 85%, at least 95%, at least 99% or about 100% of the cells in the culture, and optionally such culture (comprising such % of RG cells) may be propagated for 1 week, 2 weeks, or more than 2 weeks, including 3 weeks, 4 weeks, or longer.

The RG cells may express one or more of the markers: Brn3a, Neurofilament, Tuj 1, and Thyl, and may be characterized as Brn3a(+), Neurofilament(+), Tuj1(+), and Thyl(+).

The RG cells may be cryopreserved. The RG cells may be human.

In another aspect, the disclosure provides a composition comprising RG progenitor cells produced using a method as described herein, e.g., as described in the preceding paragraphs.

In another aspect, the disclosure provides a composition comprising RG progenitor cells, which are optionally human.

The RG progenitor cells may comprise at least 50%, at least 75%, at least 85%, at least 95%, at least 99% or about 100% of the cells in the composition.

The RG progenitor cells may express one or more of the markers: Math5, Brn3a, Brn3b, Isl1, Neurofilament, and Thyl, and thus may be characterized as Math5(+), Brn3a(+), Brn3b(+), Isl1(+), Neurofilament(+), and/or Thyl(+). The RG progenitor cells may express Math5, Brn3a, Brn3b, and Isl1, and thus may be characterized as Math5(+), Brn3a(+), Brn3b(+), and Isl1(+). The RG progenitor cells may express Brn3a, Neurofilament, and Thyl, and thus may be characterized as Brn3a(+), Neurofilament(+), and Thyl(+). Other phenotypes for RG progenitor cells are described above.

The RG progenitor cells may be cryopreserved. The RG progenitor cells may be human.

In another aspect, the invention provides a composition for use in a method of treatment of an individual in need thereof, comprising administering a composition comprising RG progenitor cells (e.g., a composition as described herein or a composition produced using a method as described herein) to said individual.

The composition may be administered to the eye, vitreous, subretinal space, or intravenously. The RG progenitor cells may be human.

In another aspect, the disclosure provides a composition comprising RG cells produced according to a method as described herein, e.g., in the preceding paragraphs.

The RG cells produced by the method may comprise at least 50%, at least 75%, at least 85%, at least 95%, at least 99% or about 100% of the cells in the composition, and optionally such composition (comprising such % of RG cells) may be propagated in culture for 1 week, 2 weeks, or more than 2 weeks, including 3 weeks, 4 weeks, or longer.

The RG cells may express one or more of the markers: Brn3a, Neurofilament, Tuj 1, and Thyl, and thus may be characterized as Brn3a(+), Neurofilament(+), Tuj1(+), and/or Thy1 (+).

The RG cells may be cryopreserved. The RG cells may be human.

In another aspect, the disclosure provides a method of treatment of an individual in need thereof, comprising administering a composition comprising RG cells, e.g., a composition as described herein such as in the preceding paragraphs or a composition produced by a method as described herein e.g., in the preceding paragraphs, to said individual.

The composition may be administered to the eye, the vitreous, the subretinal space, or intravenously.

The invention relates to a substantially pure preparation of RG progenitor cells or RG cells of human origin, preferably non-donor derived RG progenitor cells or RG cells, originating from cells not grown on a mouse fibroblast feeder platform. For example, the preparation may be 85%-95% pure. The invention relates to a method of preparing the substantially pure preparation of RG progenitor cells or RG cells of human origin which omits the need for cells derived from a mouse fibroblast feeder platform. Replacing a feeder system with the methods of the present invention produces a greater homogeneity of RG progenitor cells or RG cells, e.g., at 75% to 100% or 85% to 95%. The differentiation of the feeder-free stem cells can also occur in the absence of the introduction of exogenous inducing factors, which is a substantial improvement over the prior art. The optional addition of Noggin polypeptide, however, can accelerate differentiation of the stem cells, even though it is not essential for differentiation.

In another embodiment, the differentiation methods are feeder-free and xeno-free. Thus, in some embodiments, human cells are cultured in the absence of any media component that is derived from a non-human species.

### BRIEF DESCRIPTION OF THE DRAWINGS

FIGs. 1A and B. Gene expression during human ES cell *in vitro* differentiation. (FIG. 1A) Flow cytometry analyses of eye field progenitor markers, Pax6 and Rx1 on day 9, day 11 and day 13 of *in vitro* cell differentiation. FIG. 1B. Quantitative RT-PCR analyses of ES cell pluripotent markers in ES cells (first bar of each pair) and eye field progenitors (second bar of each pair).
FIGs. 2A and B. Flow cytometry analysis showed expression of Pax6 and Rx1 (FIG. 2A), and Otx2, Sox2 and Nestin (FIG. 2B).
FIGs. 3A and B. Expression of transcription factors that are essential for retinal ganglion cell (RGC) differentiation. (FIG. 3A) Real-time RT-PCR analysis for expression of Math5, Brn3a, Brn3b and Isl1 in ES cells (first bar of each bar pair) and retinal ganglion cell (RGC) progenitors (second bar of each bar pair) on day 35 of culture. (FIG. 3B) Quantification of the expression of Math5, Pax6 and Brn3a by immunostaining and flow cytometry.
FIG. 4. Real-time RT-PCR analysis for expression of Math5, Brn3a and Brn3b in ES cells (first bar in each triplet), early retinal ganglion cell (RGC) progenitors (second or middle bar in each triplet), and late retinal ganglion cell (RGC) progenitors (third or last bar in each triplet).
FIG. 5. Expression of Thy1 in ES cell derived mature retinal ganglion cells (RGC). Left panel, immunostaining of Thy1; Right panel, quantification of the expression of Thy1 by flow cytometry.
FIGs. 6A and B. Differentiated human ES cell derived retinal ganglion cells. (FIG. 6A) Morphology of ES cell derived retinal ganglion cells. (FIG. 6B) Cells expressed Brn3a, neurofilament and Tuj 1.
FIG. 7. Schematic of animal studies in a glaucoma rat model involving laser photocoagulation. Another animal glaucoma model involving microbead injection in the eye is described below.
FIG. 8. Quantification of retinal ganglion cell (RGC) survival at 5 weeks after intraocular pressure (IOP) elevation in control (High IOP + PBS), cell treated (High IOP + Cells) and normal rats.
FIG. 9. Timeline of differentiation from pluripotent stem cells through to retinal ganglion progenitor cells and retinal ganglion cells.

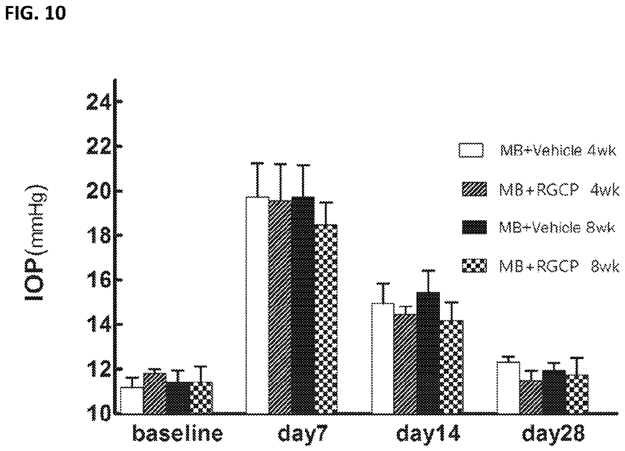

FIG. 10. Effect of RGC progenitors on intraocular pressure (IOP) in the microbead (MB) mouse glaucoma model. MB treatment consisted of one eye injection (2 µl of 15 µm microbeads, 5x10⁶/µl). The contralateral eye was used as an internal control. Cell treatment consisted of 2 µl of RGCPs (300,000 cells) by vitreous injection. Mice receiving 2 µl of medium by vitreous injection were used as the controls. MB were injected at day 0, RGCP or vehicle injection was performed on day 7, and on days 28 or 56 (4 and 8 weeks) the mice were sacrificed. RGC and retina function (pSTR and ERG, Fortune et al. IVOS, 2004, 45: 1854-1862; Smith et al. Doc Ophthalmol, 2014, 128:155-168)), and visual behavior (optokinetics) were assessed. No statistically significant difference in IOP was observed between the experimental groups indicating that the transplanted RGCP do not impact IOP and rather are working through another mechanism.

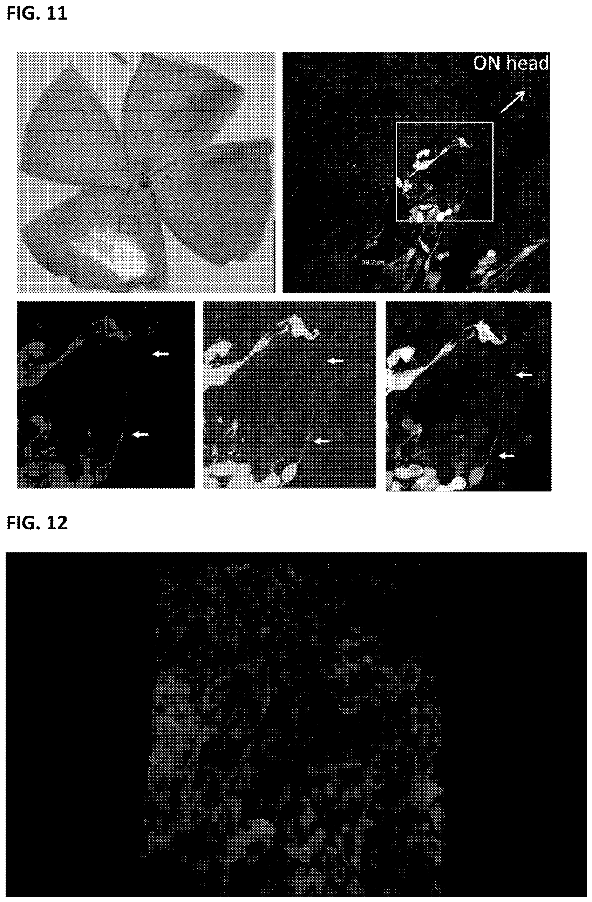

FIG. 11. Integration of transplanted retinal ganglion cell progenitors (RGCPs). Transplanted RGCPs expressed RGC marker Tuj 1 (bottom left), integrated into the retina of MB-treated mice and upon further differentiation formed extended long neurites (toward optic nerve head, bottom middle).
FIG. 12. Integration of transplanted retinal ganglion cell progenitors (RGCPs) into the host retinal ganglion layer.

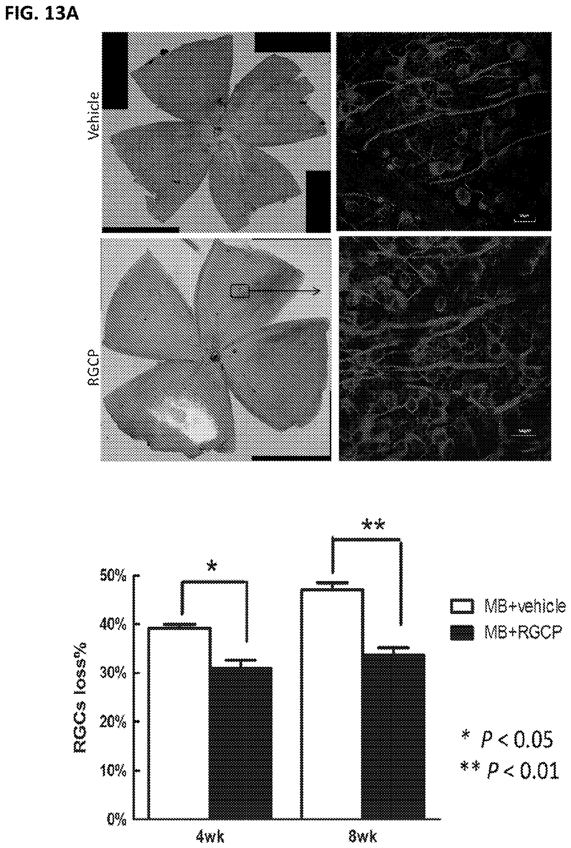

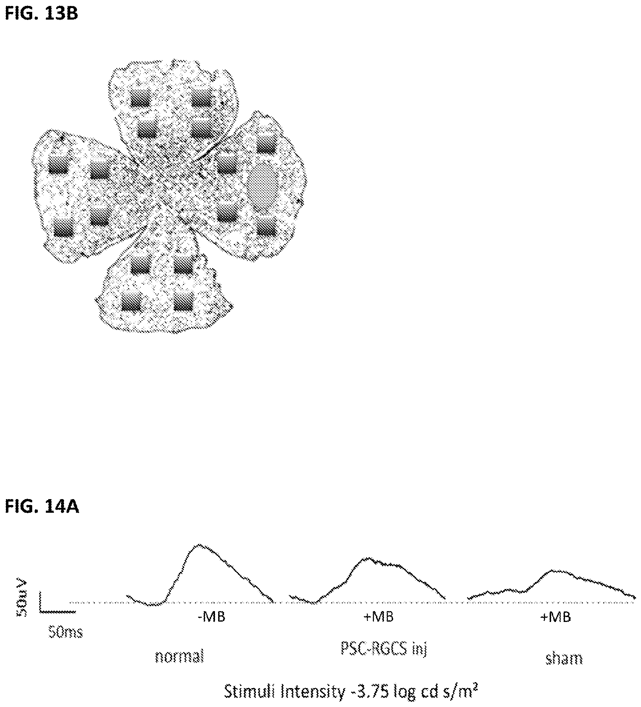

FIGs. 13A and B. Quantification of RGC loss in MB-treated mice. FIG. 13A shows that transplantation of ESC-RGCPs supports the survival of host RGCs in glaucoma mice at 4 (left) and 8 (right) weeks after RGCP injection (black bar). White bars represent mice receiving PBS injection. *p< 0.05, **p<0.01. FIG. 13B shows the regions from which the retinal ganglion layer was sampled. The oval represents the area of the RGCP transplant. Significantly, the host retinal ganglion layer was sampled in regions remote from the site of injection, and still an abatement of RGC loss was observed as a result of the RGCP injection. This suggests that a paracrine mechanism may be involved in the ability of the transplanted cells to promote survival of the endogenous, native retinal ganglion layer.

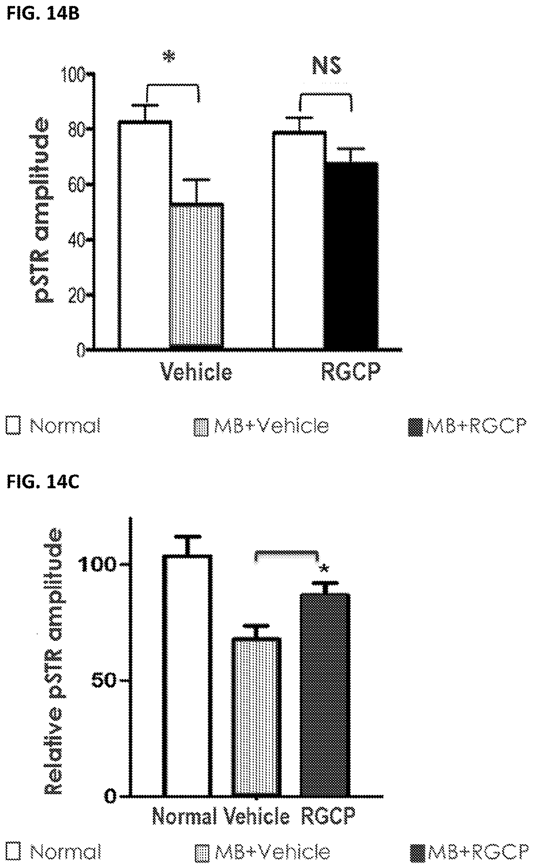

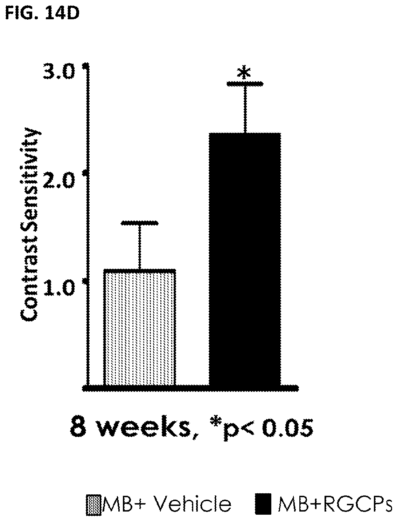

FIGs. 14A-D. Transplantation of RGCPs enhances pSTR amplitude in glaucoma mice. pSTR is a measure of RGC function. FIG. 14A shows representative waveforms of pSTR recordings for normal mice (no MB), sham treated mice (MB and vehicle injected), and ESC-RGCs treated mice (MB and PSC-RGCPs injected). FIG. 14B shows quantification of pSTR amplitude from a normal (normal), RGCP-transplanted (MB+RGCP) or vehicle-injected (MB+vehicle) glaucomatous mouse at 4 weeks post injection. Glaucomatous eyes that received PBS injections showed significantly diminished pSTR amplitude from the normal baseline, while those that received RGCP injections showed no significant reduction in pSTR amplitude compared to the baseline. FIG. 14C shows relative pSTR amplitude of these three experimental groups (normal, MB+vehicle, and MB+RGCP) and indicates the difference in relative amplitude between sham treated and RGCP treated mice is significant. FIG. 14D shows quantification of visual contrast sensitivity with OKR showing significantly enhanced visual contrast sensitivity in RGCP transplanted eyes compared to vehicle-injected eyes at 8 weeks post-injection. *p< 0.05, **p<0.01; NS: non-significant difference.

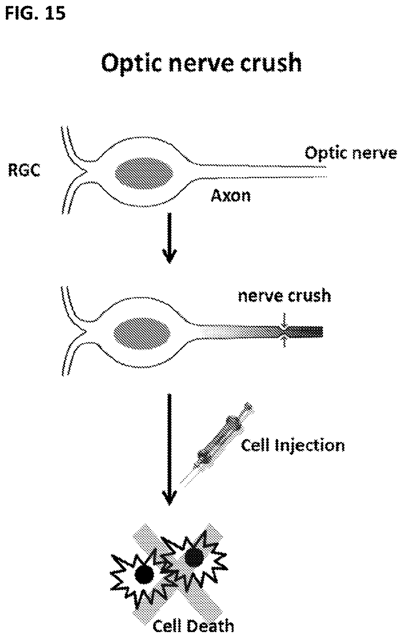

FIG. 15. Optic nerve crush model schematic.

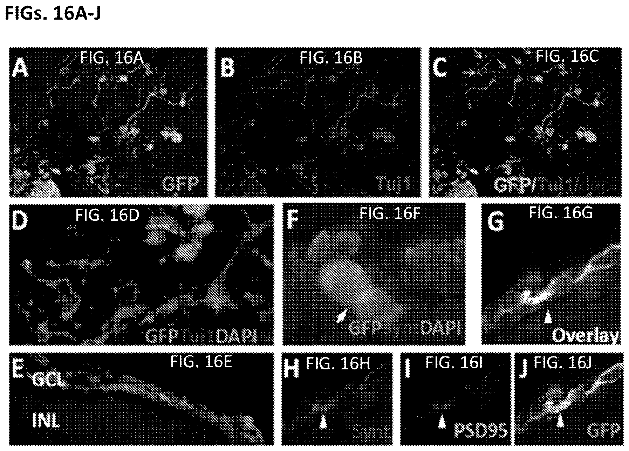

FIGs. 16A-J. RGCP repopulation, differentiation, and synapse formation in optic nerve injured mice. FIGs. 16A-C are epifluorescent images of engrafted RGCPs (GFP+) in Tuj1 immunolabeled retinal flat-mount taken from a mouse at 3 weeks after optic nerve injury. FIG. 16A shows GFP staining, FIG. 16B shows Tuj1 staining, and FIG. 16C shows GFP and Tuj1 staining. Nearly all GFP+ cells are immunolabeled positive for Tuj 1 and many bear long neurites. FIGs. 16D-E are images taken from a video of 3D light-sheet microscopy which recorded a Tuj 1-immunolabeled retinal flat-mount of a mouse that received hESC-derived RGCP transplantation. GFP+ RGCP-derived cells co-localized with RGC marker (Tuj 1) (FIG. 16D, counterstained with DAPI) and repopulated into the ganglion cell layer (GCL) (FIG. 16E). INL: inner nuclear layer. FIGs. 16F-J are representative epifluorescence confocal images taken from a retinal section (FIGs. 16F, H-J) or flat-mount (FIG. 16G) that were immunolabeled for post-synaptic marker PSD95 (FIG. 16I) and/or presynaptic marker, synaptotagmin (Synt) (FIG. 16F, stained for GFP, Synt and DAPI). Arrows point to spot areas of a cell showing positive immunolabeling. The overlay image in FIG. 16J reveals co-localization of pre- and post-synaptic markers on GFP+ cells, suggesting formation of functional synapses by grafted cells.

Table 1. Components of DMEM/F12 medium, Neurobasal™ Medium (Life Technologies), N2 Supplement, and B27 Supplement.

### DETAILED DESCRIPTION OF THE INVENTION

The invention provides methods for generating retinal ganglion (RG) cells and RG progenitor cells. These methods involve *in vitro* differentiation from earlier progenitors including pluripotent stem cells and eye field (EF) progenitors. The methods provided herein may use as a starting material any of the foregoing progenitor (including stem cell) populations.

The invention further contemplates generating RG cells and RG progenitor cells *in vitro* from primary eye field (EF) progenitors (i.e., EF progenitor cells that are obtained from a subject).

Retinal neural development *in vi*vo or *in vitro* occurs through a number of developmental stages, each of which can be defined phenotypically (e.g., by way of marker expression profile) and/or functionally. *In vitro* pluripotent stem cells differentiate into EF progenitors, which in turn differentiate into RG progenitor cells, which may comprise early RG progenitor cells and late RG progenitor cells, which in turn differentiate into mature RG cells (referred to herein as RG cells). Within the RG progenitor cells, the early RG progenitor cells may differentiate into late RG progenitor cells. The late RG progenitor cells may differentiate into mature RG cells. The early RG progenitor cells may differentiate directly into mature RG cells. The methods provided herein efficiently generate RG cells and RG progenitor cells.

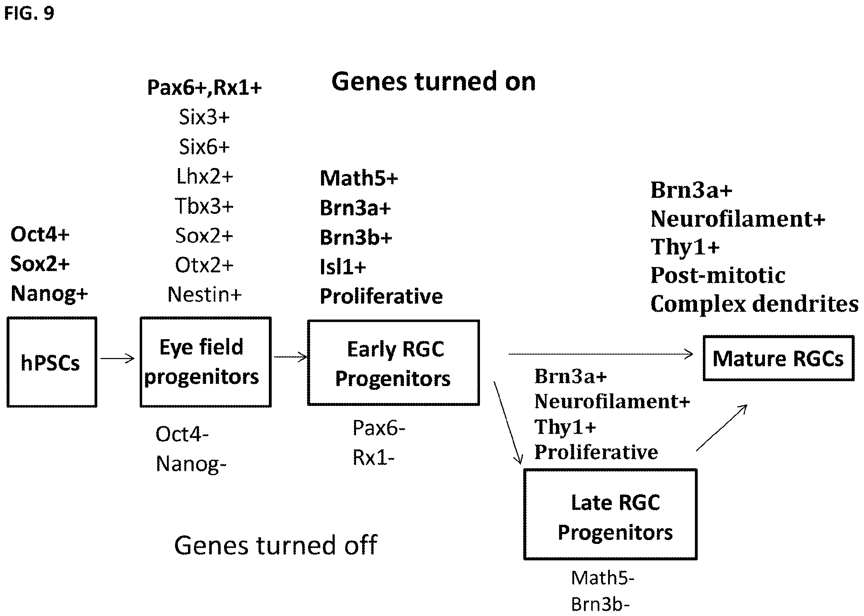

The overall developmental pathway from pluripotent stem cells to RG progenitor cell and RG cell development is schematically illustrated in FIG. 9. Also shown is the marker expression profile for each stage of development.

Progenitor cells, as used herein, refer to cells that remain mitotic and can produce more progenitor cells or can differentiate to an end fate cell lineage. The terms progenitor and precursor are used interchangeably. Cells at each of these stages will be discussed in greater detail herein.

The RG progenitors and RG cells provided herein may be used in a variety of *in vivo* and *in vitro* methods. For examples, the RG progenitor cells may be used *in vivo* to treat conditions of the retina, including glaucoma, such as open-angle glaucoma, angle-closure glaucoma, normal-tension glaucoma, congenital glaucoma, pigmentary glaucoma, secondary glaucoma, pseudoexfoliative glaucoma, traumatic glaucoma, neovascular glaucoma, and irido corneal endothelial syndrome, as well as optic nerve injury, optionally optic nerve injury associated with autoimmune disease, trauma, viral infection, and ischemic-reperfusion injury, optionally ischemic-reperfusion injury associated with stroke, diabetic retinopathy, or other condition that impairs blood supply to the eye.

The invention further provides RG progenitor cells and RG cells obtained by the methods described herein. RG progenitor cells and RG cells are obtained by *in vitro* differentiation of pluripotent stem cells or their differentiated progeny such as eye field progenitor cells. Eye field progenitor cells may themselves be obtained from *in vitro* differentiation of pluripotent stem cells, or they may be primary eye field progenitors obtained from a subject.

The invention provides populations of RG progenitor cells and populations of RG cells that have not been attained or are not attainable from primary sources. These populations may be homogenous or near homogeneous in their cell content. For example, at least 50%, at least 60%, at least 70%, at least 80%, at least 90%, at least 95%, at least 99%, or about 100% of the cells in such a population may be RG progenitor cells. As another example, at least 50%, at least 60%, at least 70%, at least 80%, at least 90%, at least 95%, at least 99%, or about 100% of the cells in such a population may be RG cells. These cells in these populations may be of a single haplotype. For example, they may be HLA-matched. These cells in these populations may be genetically identical. The present disclosure also describes a bank of HLA-typed RG progenitor cells. Such cells may be used in a variety of human subjects.

In addition, the methods provided herein can be used to generate large numbers of RG progenitor cells and RG cells that would not otherwise be possible from primary sources. For example, the methods may produce 10⁸ RG progenitor cells or 10⁸ RG cells per starting 1 million pluripotent cells.

### Differentiated (non-stem) cell types

*Eye field progenitor cells (EF progenitor cells).* EF progenitor cells are minimally defined as Pax6(+) and Rx1(+) cells. They may also be Six3(+), Six6(+), Lhx2(+), and Tbx3(+). All of these markers are transcription factors. The EF progenitor cells may also be Nestin(+) and/or Sox2(+), and/or Otx2(+). EF progenitor cells do not express Oct4 and Nanog (i.e., they are Oct4(-) and Nanog(-)). EF progenitor cells can be formed through *in vitro* differentiation of pluripotent stem cells. Such differentiation may occur in the presence of retinal induction (RI) medium, and may occur in the presence or absence of exogenous factors such as Noggin polypeptide or Noggin-like compounds.

*Retinal ganglion (RG) progenitor cells.* The differentiative methods of the invention have allowed RG development to be dissected even further than was previously possible, thereby revealing two RG progenitor stages. The first stage to develop from EF progenitor cells is the early RG progenitor cell stage. These cells are defined by expression of one or more or all of Math5, Brn3a, Brn3b, and Isl1 (i.e., Math5(+) and/or Brn3a(+) and/or Brn3b(+) and/or Isl1(+)) and no or undetectable expression of Pax6 and Rx1 (i.e., Pax6(-) and Rx1(-)). These RG progenitors may be Math5(+), or Math5(+), Brn3a(+),or Math5(+), Brn3a(+), Brn3b(+), or Math5(+), Brn3a(+), Brn3b(+), Isl1(+). These early RG progenitor cells differentiate further into late RG progenitor cells. These cells are defined by the expression of one or more or all of Brn3a, Neurofilament, and Thy1 (i.e., Brn3a(+) and/or Neurofilament(+) and/or Thy1(+)) and no or undetectable expression of Math5, Brn3b, and Isl1 (i.e., Math5(-), Brn3b(-) and Isl1(-)). These RG progenitors may be Brn3a(+), Neurofilament(+) and Thyl(+). These later RG progenitor cells then differentiate into RG cells. Early RG progenitor cells can also differentiate directly into RG cells, for example when cultured in RGC differentiation medium. RG progenitor cells are proliferative.

*Retinal ganglion (RG) cells.* RG cells are minimally defined by the expression of one or more or all of Neurofilament, Tuj 1, Thy1 and Brn3a (i.e., Neurofilament(+) and/or Tuj 1(+) and/or Thy1(+) and/or Brn3a(+)). RG cells may be Brn3a(+), Neurofilament(+), Tuj 1(+) and optionally Thyl(+). RG cells are post-mitotic. RG cells may have more dendrites, more complex dendrites, and longer dendrites than RG progenitor cells.

### Culture methods

*Pluripotent stem cell maintenance culture.* The pluripotent stem cells may be maintained and thus propagated on a feeder-free system, optionally on a matrix. Suitable matrices include those comprising or consisting of laminin, fibronectin, vitronectin, proteoglycan, entactin, collagen, collagen I, collagen IV, collagen VIII, heparan sulfate, Matrigel™ (a soluble preparation from Engelbreth-Holm-Swarm (EHS) mouse sarcoma cells), CellStart (a human basement membrane extract), or any combination thereof. In some embodiments, the matrix comprises, consists of, or consists essentially of Matrigel™. Matrigel™ is available from commercial sources such as BD Biosciences. Laminin, including recombinant human laminin, may be used as an alternative to Matrigel™.

In an exemplary method, the pluripotent stem cells are maintained in their undifferentiated state under feeder-free conditions on Matrigel™ or laminin. The cells are typically passaged or frozen at about 80-90% confluency. Passaging can be performed using enzymatic (e.g., dispase) or non-enzymatic (e.g., PBS-based dissociation buffer, Invitrogen) means. In some embodiments, the pluripotent stem cells may be cultured under xeno-free conditions, and in some instances may include culture in the presence of serum supplements such as N2 serum supplement.

*Pluripotent stem cell differentiation culture.* The pluripotent stem cells may be directly differentiated, intending that their differentiation into RG cells or RG progenitor cells does not involve differentiation into non-ganglion cell lineages, as may occur for example in an embryoid body (EB). Thus, the methods provided herein do not require and preferably avoid EB formation. This is an improvement over prior art methods.

RG progenitor cells or RG cells may be generated by suspension culture involving the formation of neurospheres, or attached to a matrix such as Matrigel™ or laminin.

The following is an exemplary process for differentiating pluripotent stem cells into RG progenitor cells and RG cells.

The pluripotent stem cells are first differentiated into EF progenitor cells by culturing the pluripotent stem cells in retinal induction (RI) medium and neural differentiation (ND) medium.

RI medium is added to pluripotent stem cells that are at about 15-20% confluency, at day 0. RI medium comprises a basal medium such as DMEM/F12 or a medium comprising the components of DMEM/F12, D-glucose, antibiotics such as penicillin and streptomycin, one or more serum supplements such as N2 supplement and B27 supplement, non-essential amino acids, insulin, and optionally exogenous differentiation factors such as Noggin polypeptide or Noggin-like factors as described below.

The components of DMEM/F12, N2 Supplement, and B27 Supplement are provided in Table 1.

Culture in RI medium may occur for about 5 days. A complete media change is performed on days 1-4.

After about 4 days (e.g., at or around day 4, 5, or 6), the medium is changed to neural differentiation (ND) medium. ND medium comprises a basal medium such as Neurobasal™ Medium (Life Technologies) or a medium comprising the components of Neurobasal™ Medium, D-glucose, antibiotics such as penicillin and streptomycin, GlutaMAX™, one or more serum supplements such as N2 supplement and B27 supplement, non-essential amino acids, and optionally exogenous differentiation factors such as Noggin polypeptide or Noggin-like factors as described below.

The components of Neurobasal™ Medium (Life Technologies) are provided in Table 1.

GlutaMAX™ is L-alanyl-L-glutamine which is a stabilized form of L-glutamine. It experiences less degradation in culture, thereby reducing the ammonia build-up that can occur in long-term cultures.

The cells are cultured for about another 8 days (days 6-13) in ND medium. The medium is changed regularly (e.g., about every 2 days). During this culture period, the cells grow as colonies, with the edge cells becoming flat and large while the more centrally located cells being smaller and forming compact cell clusters.

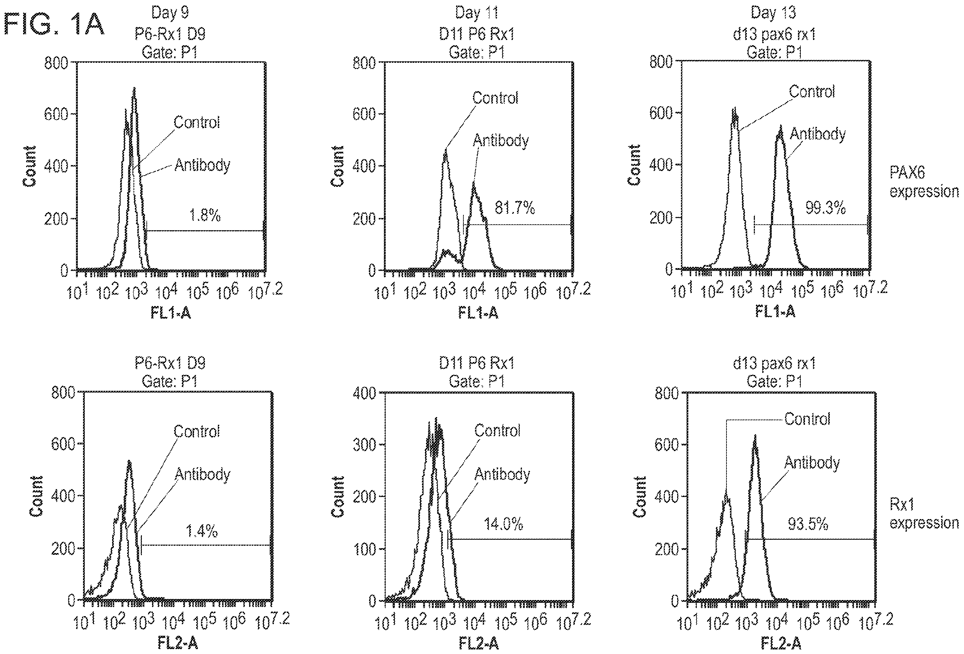

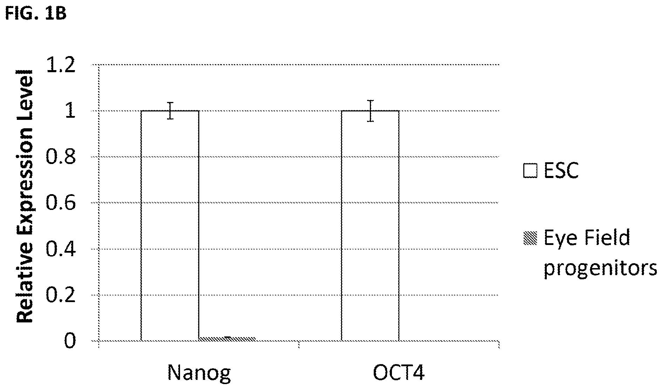

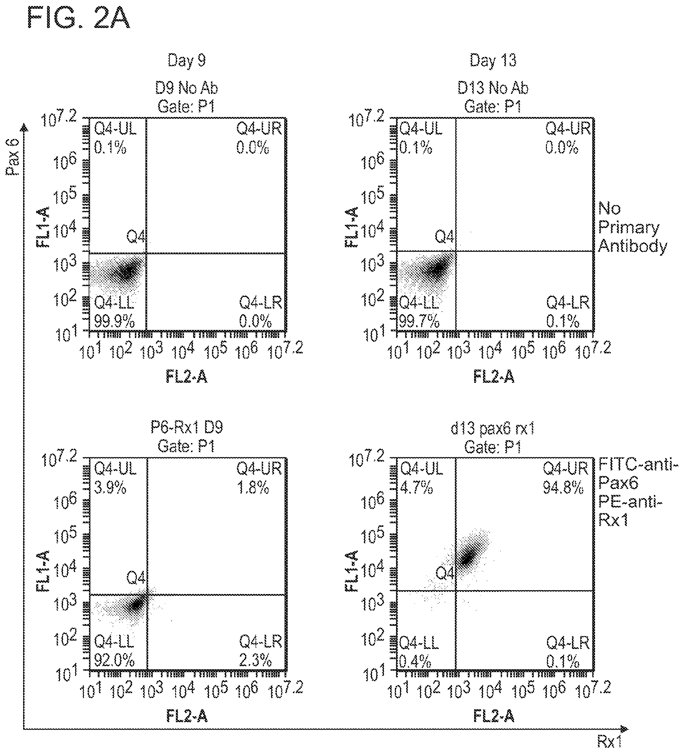

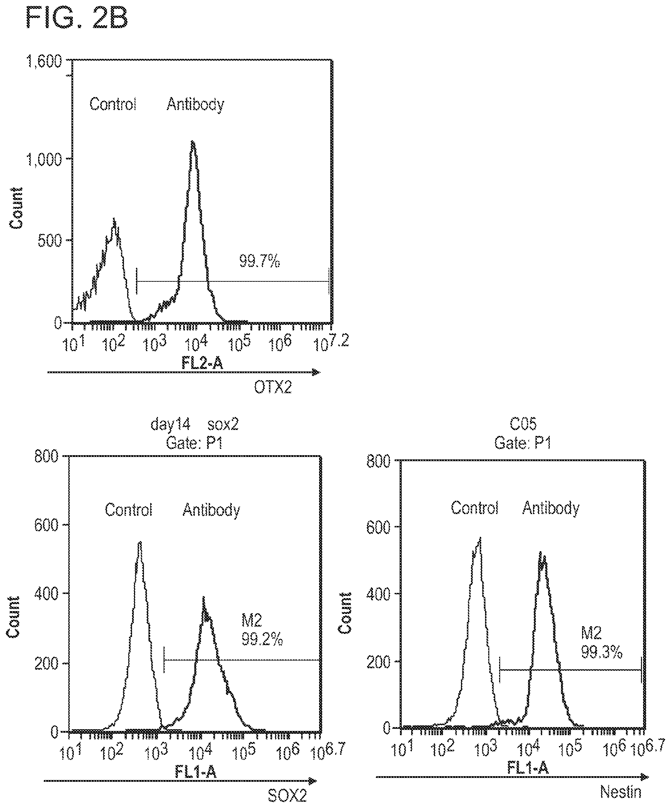

At about days 10-11, the cells start to express Pax6, followed by the onset of Rx1 expression on day 12 (FIG. 1A). RT-PCR analysis showed loss of Oct4 and Nanog expression (FIG. 1B). On day 13, over 90% cells co-express Pax6 and Rx1 (FIG. 2A) as quantified flow cytometry, and over 99% of cells express Nestin, Otx2 and Sox2 (FIG. 2B). These results indicate that the pluripotent stem cells have differentiated into EF progenitor cells.

At about day 14 (e.g., at or around day 11, 12, 13, 14, or 15), the medium is changed to ganglion cell (GC) medium. GC medium comprises a basal medium such as Neurobasal™ Medium (Life Technologies) or a medium comprising the components of Neurobasal™ Medium, D-glucose, one or more antibiotics such as penicillin and streptomycin, GlutaMAX™, one or more serum supplements such as N2 supplement and B27 supplement (formula 080085-SA), and one or more of including all of forskolin, BDNF and CNTF. The cells continue to be cultured in GC medium until about day 20.

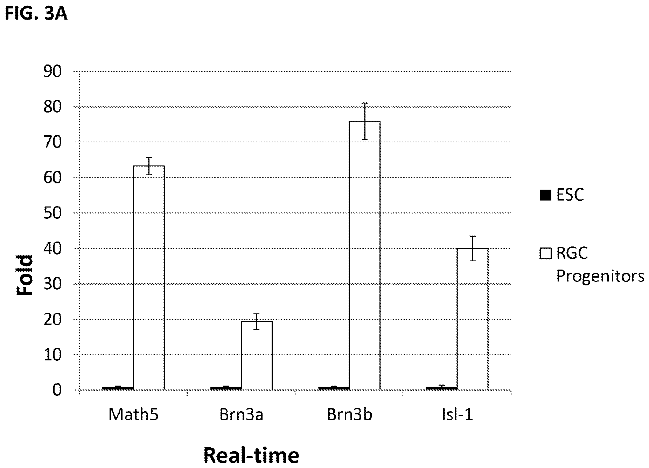

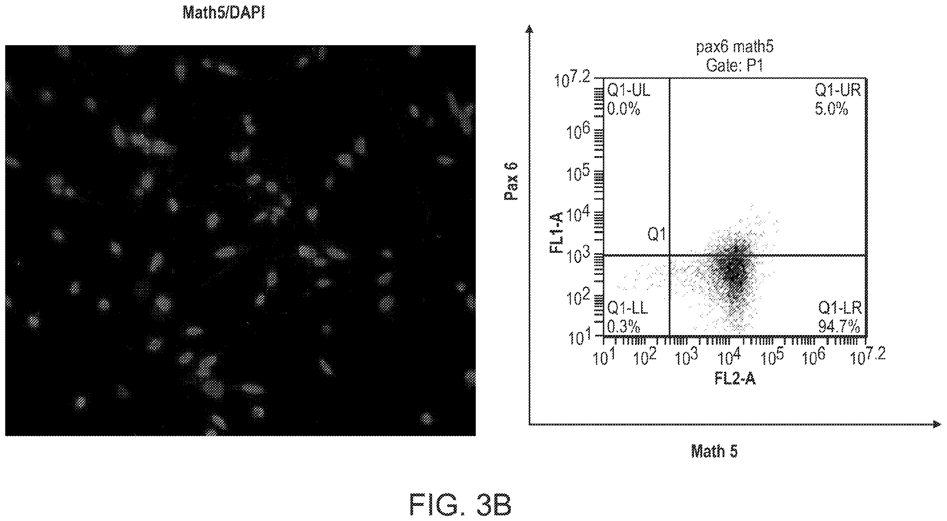

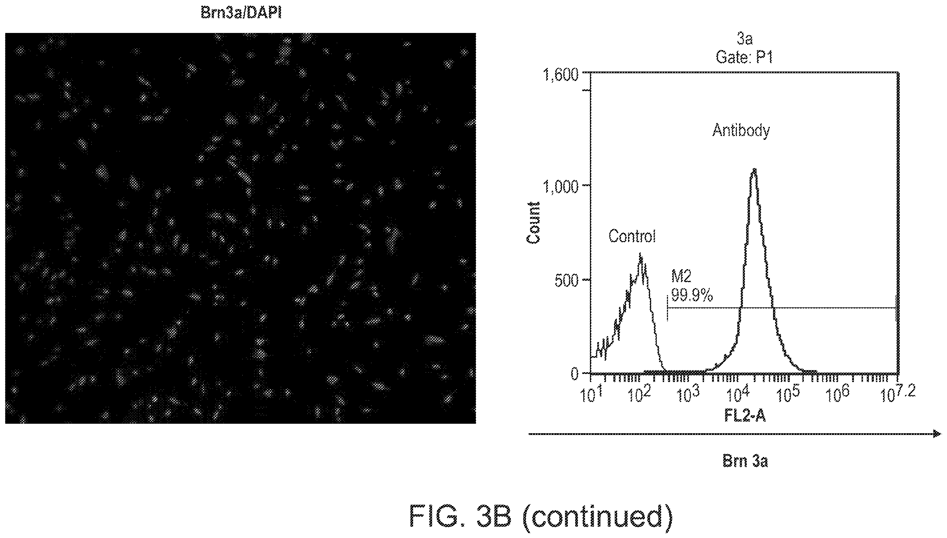

At about day 20, cells are lifted from the growth surface using a cell scraper, and mechanically fragmented into clusters in GC medium. The cell clusters are transferred to 100 mm ultra-low attachment culture dishes. Cell clusters round up and form individual spheres in this suspension culture. The GC medium is changed every 3 days. At about day 35, real-time RT-PCR showed a strong up-regulation of the expression of Math5, Brn3a, Brn3b and Isl1 (FIG. 3A). Flow cytometry showed that >99% cells expressed transcription factors Math5 and Brn3a. About 94% Math5(+) cells stained negative for Pax6 (FIG. 3B). These results indicate the presence of early RG progenitor cells.

Between about days 36-60, the neural spheres are cultured in GC medium, with media changes about every 3-4 days.

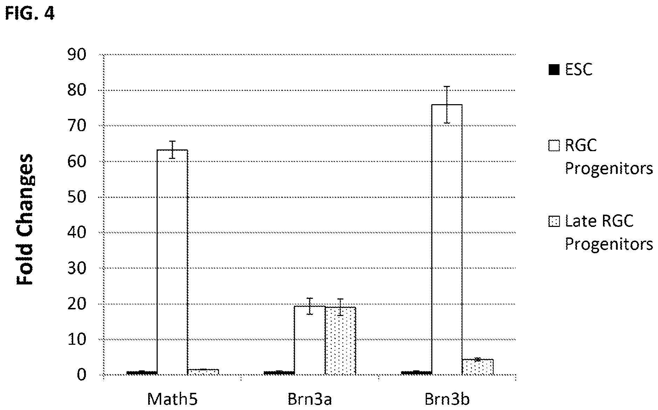

At about day 60, real-time RT-PCR showed continued expression of Brn3a. The expression levels of Math5 and Brn3b were greatly down-regulated (FIG. 4). Flow cytometry showed 95.3% of cells co-expressed Brn3a and Neurofilament (data not shown). These results indicate the presence of late RG progenitor cells.

*Differentiation of RG progenitor cells to form RG cells.* RG progenitor cells are dissociated into single cells using Accutase® cell detachment solution. Cells are plated on poly-D-lysine/laminin coated plates at the density of about 10-40/cm² in retinal ganglion cell (RGC) differentiation medium. RCG differentiation medium comprises a basal medium such as Neurobasal™ Medium (Life Technologies) or a medium comprising the components of Neurobasal™ Medium, D-glucose, antibiotics such as penicillin and streptomycin, GlutaMAX™, one or more serum supplements such as B27 supplement (formula 080085-SA), insulin, one or both of BDNF and CNTF, and one or more including all of forskolin, cAMP and DAPT or other Notch inhibitor. The components of Neurobasal™ Medium are provided in Table 1. The media was changed about every 2 days.

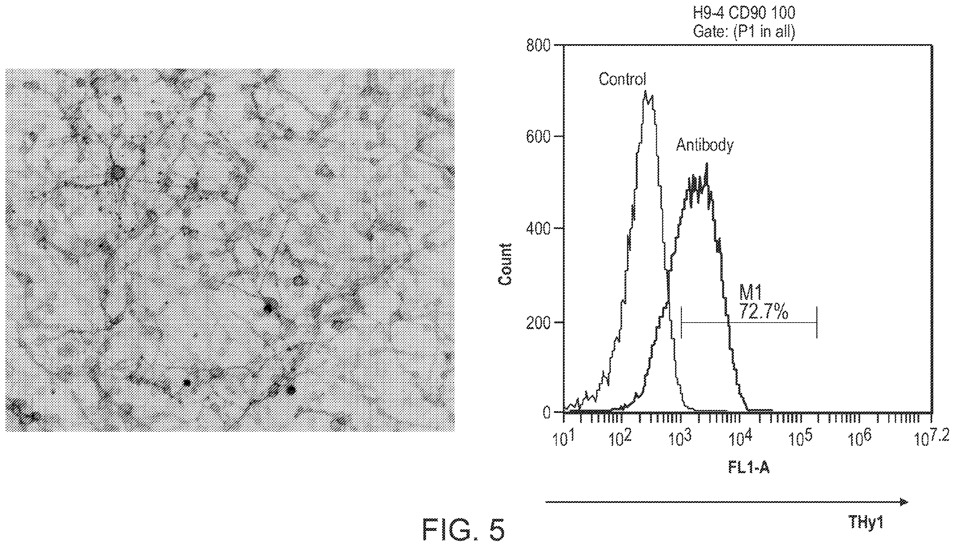

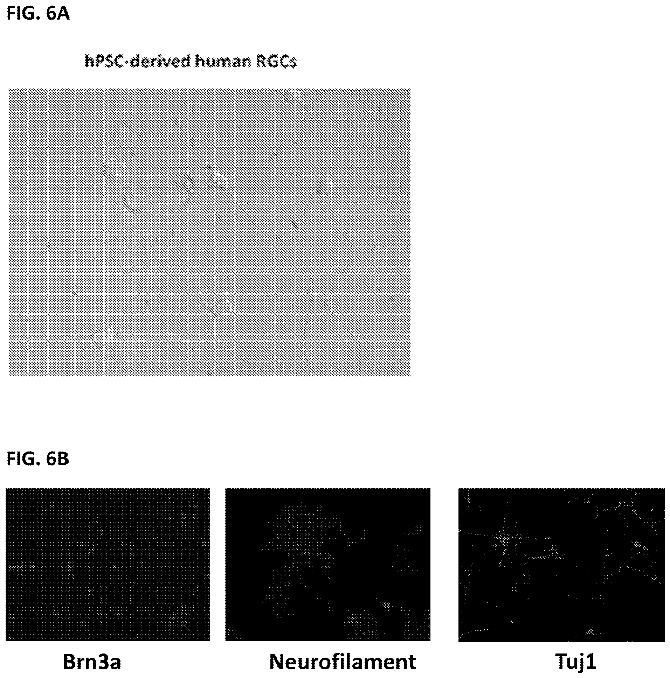

After 2 weeks, about 72% of cells stained positive for Thy1 (FIG. 5). Cells had dendrites and axons (FIG. 6A). Immunostaining showed expression of Brn3a, neurofilament and Tuj 1 (FIG. 6B). These results indicate the presence of RG cells.

*Cryopreservation of RG progenitors and RG cells.* Early RG progenitor cells, late RG progenitor cells, and RG cells can be frozen/cryopreserved in an animal-free cryopreservation buffer, such as Cryostor CS10, as neurospheres.

### Cell culture media

In embodiments of the invention, the cells are stored, proliferated or differentiated in various cell culture media. These media are described in greater detail below.

*Retinal induction (RI) medium.* Retinal induction (RI) medium is used to differentiate pluripotent stem cells into EF progenitor cells. Retinal induction (RI) medium comprises a basal medium such as DMEM/F12 or a medium comprising the components of DMEM/F12 (as shown in Table 1), D-glucose (optionally between 0-10 mg/ml, 2.5-7.5 mg/ml, 3-6 mg/ml, 4-5 mg/ml, or about 4.5 mg/ml), one or more antibiotics such as penicillin (optionally between 0-100 units/ml or about 100 units/ml) and streptomycin (optionally between 0-100 µg/ml or about 100 µg/ml), one or more serum supplements such as N2 supplement (optionally at about 0.1 to 5% or about 0.1 to 2% or about 1% (v/v) and B27 supplement (optionally about 0.05-2.0% or about 0.2% (v/v), MEM non-essential amino acids solution (optionally about IX concentration made from a stock, approximately 0.1 mM glycine), and optionally insulin (optionally 5-50 µg/ml or about 20 µg/ml). The RI medium may comprise Noggin polypeptide at a concentration of between about 5-100 ng/ml or about 10-100 ng/ml or about 10-500 ng/ml or about 50 ng/ml. The retinal induction medium may comprise a BMP signaling inhibitor, optionally selected from the group consisting of Noggin polypeptide, dorsomorphin, LDN-193189, and any combination thereof. RI medium may include insulin, Noggin polypeptide, or insulin and Noggin polypeptide.

Noggin polypeptide is not necessary although increased differentiation into EF progenitors cells (as indicated by increased expression of EF transcription factors) is observed when Noggin polypeptide is present. Noggin polypeptide can be substituted with other moieties having similar activity, including SB431542.

Noggin polypeptide is a secreted BMP inhibitor that reportedly binds BMP2, BMP4, and BMP7 with high affinity to block TGFβ family activity. SB431542 is a small molecule that reportedly inhibits TGFβ/Activin/Nodal by blocking phosphorylation of ACTRIB, TGFβR1, and ACTRIC receptors. SB431542 is thought to destabilize the Activin- and Nanog-mediated pluripotency network as well as suppress BMP-induced trophoblast, mesoderm, and endodermal cell fates by blocking endogenous Activin and BMP signals.

It is expected that agents having one or more of the aforementioned activities could replace or augment the functions of one or both of Noggin polypeptide and SB431542, e.g., as they are used in the context of the disclosed methods. For example, Noggin polypeptide and/or the small molecule SB4312542 could be replaced or augmented by one or more inhibitors that affect any or all of the following three target areas: 1) preventing the binding of the ligand to the receptor; 2) blocking activation of receptor (e.g., dorsomorphin), and 3) inhibition of SMAD intracellular proteins/transcription factors. Exemplary potentially suitable factors include the natural secreted BMP inhibitors Chordin (which blocks BMP4) and Follistatin (which blocks Activin), as well as analogs or mimetics thereof. Additional exemplary factors that may mimic the effect of Noggin polypeptide include use of dominant negative receptors or blocking antibodies that would sequester BMP2, BMP4, and/or BMP7. Additionally, with respect to blocking receptor phosphorylation, dorsomorphin (or Compound C) has been reported to have similar effects on stem cells. Inhibition of SMAD proteins may also be effected using soluble inhibitors such as SIS3 (6,7-Dimethoxy-2-((2E)-3-(1-methyl-2-phenyl-1H-pyrrolo[2,3-b]pyridin-3-yl-prop-2-enoyl))-1,2,3,4-tetrahydroisoquinoline, Specific Inhibitor of Smad3, SIS3), overexpression of one or more of the inhibitor SMADs (e.g., SMAD6, SMAD7, SMAD10) or RNAi for one of the receptor SMADs (SMAD1, SMAD2, SMAD3, SMAD5, SMAD8/9). Another combination of factors expected to be suitable for generating neural progenitors comprises a cocktail of Leukemia Inhibitory Factor (LIF), GSK3 inhibitor (CHIR 99021), Compound E (y secretase inhibitor XXI) and the TGFβ inhibitor SB431542 which has been previously shown to be efficacious for generating neural crest stem cells (Li et al., Proc Natl Acad Sci U S A. 2011 May 17;108(20):8299-304). Additional exemplary factors may include derivatives of SB431542, e.g., molecules that include one or more added or different substituents, analogous functional groups, etc. and that have a similar inhibitory effect on one or more SMAD proteins. Suitable factors or combinations of factors may be identified, for example, by contacting pluripotent cells with said factor(s) and monitoring for adoption of EF progenitor cell phenotypes, such as characteristic gene expression (including expression of the markers described herein, expression of a reporter gene coupled to an EF progenitor cell promoter, or the like) or the ability to form a cell type disclosed herein such as retinal neural progenitor cells, RG progenitor cells or RG cells.

Preferably the cells are treated with or cultured in a retinal induction medium prior to culture with a neural differentiation medium.

*Neural differentiation (ND) medium.* Neural differentiation (ND) medium is used to differentiate pluripotent stem cells into eye field (EF) progenitor cells. ND medium comprises a basal medium such as Neurobasal™ Medium (Life Technologies) or medium comprising components of Neurobasal™ Medium as provided in Table 1, D-glucose (optionally between 0-10 mg/ml, 2.5-7.5 mg/ml, 3-6 mg/ml, 4-5 mg/ml, or about 4.5 mg/ml), one or more antibiotics such as penicillin (optionally between 0-100 units/ml or about 100 units/ml) and streptomycin (optionally between 0-100 µg/ml or about 100 µg/ml), GlutaMAX™ (optionally IX, approximately 2 mM), one or more serum supplements such as N2 supplement (optionally 0.1-5% v/v or 0.1-2% v/v, including 1% v/v) and B27 supplement (optionally 0.05-2% v/v, including 2% v/v), MEM non-essential amino acid solution (optionally IX concentration from a stock solution, or approximately 0.1 mM final concentration), and optionally Noggin polypeptide (optionally 10-500 ng/ml, including 50 ng/ml).

*Ganglion cell (GC) medium.* Ganglion cell (GC) medium is used to differentiate pluripotent stem cells into retinal ganglion (RG) progenitor cells, and more specifically to differentiate eye field progenitor cells into retinal ganglion (RG) progenitor cells. GC medium comprises a basal medium such as Neurobasal™ Medium (Life Technologies) or a medium comprising the components of Neurobasal™ Medium as provided in Table 1, D-glucose (optionally between 0-10 mg/ml, 2.5-7.5 mg/ml, 3-6 mg/ml, 4-5 mg/ml, or about 4.5 mg/ml), one or more antibiotics such as penicillin (optionally between 0-100 units/ml or about 100 units/ml) and streptomycin (optionally between 0-100 µg/ml or about 100 µg/ml), GlutaMAX™ (optionally IX, or approximately 2 mM), one or more serum supplements such as N2 supplement (optionally 0.1-5% v/v or 0.1-2% v/v, including 1% v/v) and B27 supplement (formula 080085-SA) (optionally 0.5-2% v/v, including 2% v/v), and one or more of forskolin (optionally 1-20 µM, and including 5 µM), BDNF (optionally 1-200 ng/ml, and including 10 ng/ml), and CNTF (optionally 1-200 ng/ml, and including 10 ng/ml).

*Retinal Ganglion Cell (RGC) differentiation medium.* RGC differentiation medium is used to differentiate pluripotent stem cells into retinal ganglion (RG) cells, and more specifically to differentiate RG progenitor cells into RG cells. RGC differentiation medium comprises a basal medium such as Neurobasal™ Medium (Life Technologies) or a medium comprising the components of Neurobasal™ Medium as provided in Table 1, D-glucose (optionally between 0-10 mg/ml, 2.5-7.5 mg/ml, 3-6 mg/ml, 4-5 mg/ml, or about 4.5 mg/ml), one or more antibiotics such as penicillin (optionally between 0-100 units/ml or about 100 units/ml) and streptomycin (optionally between 0-100 µg/ml or about 100 µg/ml), one or more serum supplements such as B-27 supplement (formula 080085-SA) (optionally at about 0.05 to 5.0% , about 1.5 to 2.5% or about 2% (v/v)), GlutaMAX™ (optionally IX, or about 2 mM), and one or more including all of forskolin (optionally 1-20 µM, and including 5 µM), BDNF (optionally 1-200 ng/ml or 5-50 ng/ml or 5-25 ng/ml or about 10 ng/ml), CNTF (optionally 1-200 ng/ml or 5-50 ng/ml or 5-25 ng/ml or about 10 ng/ml), cAMP (optionally 1-500 ng/ml or 10-250 ng/ml or 5-150 ng/ml or about 100 ng/ml), DAPT (optionally 1-100 µM or 1-20 µM), and optionally retinoic acid (optionally 0.5-20 µM or 1-5 µM). BDNF may be human BDNF. CTNF may be human CTNF. The RGC differentiation medium may comprise one or both of BDNF and CNTF. The RGC differentiation medium may comprise one or more including all of forskolin, cAMP, and DAPT or other Notch inhibitor. The RGC differentiation medium may comprise insulin, including human insulin (optionally about 5-50 µg/ml or about 20 µg/ml).

The components of DMEM/F12, Neurobasal™ Medium (Life Technologies), N2 serum supplement, and B27 serum supplement and B27 serum supplement (formula 080085-SA) are provided in Table 1. It is to be understood that the invention contemplates the use of these particular media and supplements or media or supplements comprising, consisting essentially of, or consisting of these components.

### Cell preparations and purity thereof

As used herein, the term preparation, particularly as it relates to a cell preparation, is used interchangeably with the terms population and composition. For brevity, aspects and embodiments may be described in terms of a preparation but it is to be understood that such descriptions apply equally to populations and compositions. It is to be understood that the preparation may comprise other non-cellular substituents.

As used throughout this disclosure, a level of purity of a cell preparation may be quantified as the proportion of cells in a preparation that express one or more markers of a desired cell type (including those markers identified in this application or others known in the art) relative to the total number of cells in the preparation. Purity may be determined by detecting and enumerating cells that express and/or cells that do not express one or more markers of interest, and optionally enumerating total cell number. Exemplary methods that may be used to enumerate cells include fluorescence activated cell sorting (FACS), immunohistochemistry, in situ hybridization, and other suitable methods known in the art. Typically such an analysis excludes non-viable cells in the preparation.

As used herein, a majority of cells means at least 50%, and depending on the embodiment may include at least 60%, at least 70%, at least 80%, at least 90%, at least 95%, or about 100% of cells.

The disclosure provides substantially pure (or homogeneous) preparations of various cell populations based on the ability of the disclosed methods to directly differentiate progenitor cells such as pluripotent stem cells. As used herein, directed differentiation intends that the progenitor cell population differentiates into or towards a desired lineage, due in part to the factors or other stimuli provided to such progenitor cells, thereby avoiding differentiation into other undesired, and thus potentially contaminating, lineages. The methods provided herein drive differentiation of for example pluripotent stem cells to eye field progenitors without generating embryoid bodies (EB). EBs, as described below, are three dimensional cell clusters that can form during differentiation of pluripotent stem cells including embryonic stem (ES) cells, and that typically contain cells, including progenitors, of mesodermal, ectodermal and endodermal lineages. The three dimensional nature of the EB may create a different environment, including different cell-cell interactions and different cell-cell signaling, than occurs in the non-EB based methods described herein. In addition, cells within EBs may not all receive a similar dose of an exogenously added agent, such as a differentiation factor present in the surrounding medium, and this can result in various differentiation events and decisions during development of the EB.

In contrast, the culture methods of the invention do not require and preferably avoid EB formation. Instead, these methods culture cells in conditions that provide the cells with equal contact with the surrounding medium, including factors in such medium. The cells may grow as a monolayer or near monolayer attached to a culture surface, as an example.

The ability of all or a majority of the progenitor cells to be in contact with their surrounding medium and thus the factors in such medium to an approximately equal degree results in those progenitor cells differentiating at similar times and to similar degrees. This similar differentiation timeline for a population of progenitor cells indicates that such cells are synchronized. The cells may be cell cycle synchronized in some instances also. Such synchronicity results in populations of cells that are homogeneous or near homogeneous in their cellular make-up. As an example, the methods described herein can produce cellular populations wherein at least 50%, at least 60%, at least 70%, at least 80%, at least 90%, at least 95%, or about 100% of cells are a particular cell of interest. The cell of interest may be defined phenotypically, for example by intracellular or extracellular marker expression. The cell of interest may be an eye field progenitor cell, an RG progenitor cell (whether early or late), or an RG cell.

As used herein, a "substantially pure preparation of cells" refers to a preparation of cells wherein the cells are at least 60% pure. Thus, at least 60% of the cells in the preparation express one or more markers of the particular cell type (including those markers identified in this application or others known in the art). The cells may be at least 65% pure or at least 70% pure. The cells may be about more than 70% pure including at least 75%, 80%, 85% or 90% pure.

Thus, for example, a "substantially pure preparation of RG cells" refers to a preparation of cells wherein at least 60% of the cells in the preparation express one or more markers of RG cells (including those markers identified in this application or others known in the art). At least 70% of the cells in the preparation may express one or more markers of RG cells, in which case the preparation is 70% pure. At least 85% of the cells in the preparation may express one or more markers of RG cells, in which case the preparation is 85% pure. At least 95% of the cells in the preparation may express one or more markers of RG cells, in which case the preparation is 95% pure. In some embodiments, the preparation may be about 85% to 95% pure.

As another example, a "substantially pure preparation of RG progenitor cells" refers to a preparation of cells wherein at least 60% of the cells in the preparation express one or more markers of RG progenitor cells (including those markers identified in this application or others known in the art). At least 70% of the cells in the preparation may express one or more markers of RG progenitor cells, in which case the preparation is 70% pure. At least 85% of the cells in the preparation may express one or more markers of RG progenitor cells, in which case the preparation is 85% pure. At least 95% of the cells in the preparation may express one or more markers of RG progenitor cells, in which case the preparation is 95% pure. In some embodiments, the preparation may be about 85% to 95% pure.

In certain embodiments of this disclosure, cells including RG progenitor cells and RG cells have a mean terminal restriction fragment length (TRF) that is longer than 7 kb, 7.5 kb, 8 kb, 8.5 kb, 9 kb, 9.5 kb, 10 kb, 10.5 kb, 11 kb, 11.5 kb or even 12 kb. Methods for measuring mean terminal restriction fragment length (TRF) are known in the art. See for example Kimura et al. Nat. Protocol, 5(9):1596-1607, 2010.

### Cell marker expression as a function of developmental stage

The expression of markers such as Pax6, Rx1, Six3, Six6, Lhx2, Tbx3, Sox2, Chx10, Math5, Brn3 including Brn3a and Brn3b, Isl1, Neurofilament, Tuj1, Thy1, Nestin, Otx2, Nanog, Oct4, may be assessed at the protein and/or mRNA level (see Fischer AJ, Reh TA, Dev Neurosci. 2001:23(4-5):268-76; Baumer et al., Development. 2003 Jul;130(13):2903-15; Swaroop et al., Nat Rev Neurosci. 2010 Aug;11(8):563-76; Agathocleous and Harris, Annu. Rev. Cell Dev. Biol. 2009. 25:45-69; Beby F, Lamonerie T, Exp. Eye Res. 2013; 111, 9-16).

The markers are generally human, e.g., except where the context indicates otherwise. The cell markers can be identified using conventional immunocytochemical methods or conventional PCR methods which techniques are well known to those of ordinary skill in the art.

### Pluripotent stem cells

As used herein, the term "pluripotent stem cells" are cells that are: (a) capable of inducing teratomas when transplanted in immunodeficient (SCID) mice; (b) capable of differentiating to cell types of all three germ layers (e.g., ectodermal, mesodermal, and endodermal cell types); and (c) express one or more markers of pluripotency (e.g., Oct 4, alkaline phosphatase, SSEA-3 surface antigen, SSEA-4 surface antigen, nanog, TRA-1-60, TRA-1-81, SOX2, REX1, etc). In certain embodiments, pluripotent stem cells express one or more markers selected from the group consisting of OCT-4, alkaline phosphatase, SSEA-3, SSEA-4, TRA-1-60, and TRA-1-81.

Pluripotent stem cells include embryonic stem (ES) cells, embryo-derived stem cells, embryonic germ (EG) cells, embryonic carcinoma (EC) cells, induced pluripotent stem (iPS) cells, and stimulus-triggered acquisition of pluripotency (STAP) cells. Exemplary pluripotent stem cells can be generated using any variety of methods known in the art. As will be recognized in the art, embryoid bodies can be formed from ES cells or EG cells, among others. As will also be recognized in the art, ES cells may be generated without embryo destruction. For example, ES cells may be generated from parthenogenetic ova (egg cells) which are non-embryos, ES cells may be generated from non-viable embryos including those that are deliberately engineered to be non-viable (e.g., not capable of implantation), and ES cells may be generated from single blastomeres of an embryo including a non-viable embryo.

Pluripotent stem cells may be modified, including genetically modified, to increase longevity, potency, and/or homing, and/or to prevent or reduce alloimmune responses, and/or to deliver a desired factor to the differentiated progeny of the pluripotent stem cells, and/or to express a desired factor in differentiated progeny of the pluripotent stem cells.

The pluripotent stem cells can be from any species. Various types of pluripotent stem cells, including ES cells and iPS cells, have been generated from mouse, human and other sources. Accordingly, the pluripotent stem cells used in the present methods may be obtained from any species including human, non-human primates, rodents (mice, rats), ungulates (cows, sheep, etc.), dogs (domestic and wild dogs), cats (domestic and wild cats such as lions, tigers, cheetahs), rabbits, hamsters, goats, elephants, panda (including giant panda), pigs, raccoon, horse, zebra, marine mammals (dolphin, whales, etc.) and the like. In certain embodiments, the species is an endangered species. In certain embodiments, the species is a currently extinct species.

*Embryonic stem (ES) cells.* Embryonic stem cells, regardless of their source or the particular method used to produce them, are cells that (i) are able to differentiate into cells of all three germ layers, (ii) express at least Oct 4 and alkaline phosphatase, and (iii) are able to produce teratomas when transplanted into immunodeficient animals. The term includes cells derived from the inner cell mass (ICM) of human blastocysts or morulae, including those that have been serially passaged as cell lines; cells derived from a zygote, a blastomere (e.g., of a cleavage stage or morula stage embryo), or a blastocyst-staged mammalian embryo, produced by the artificial fusion of a sperm and egg cell, fertilization of an egg cell with sperm, nuclear transfer such as somatic cell nuclear transfer (SCNT), parthenogenesis, androgenesis, or through reprogramming of chromatin and subsequent incorporation of the reprogrammed chromatin into a cell.

The term ES cells does not infer, and should not be inferred to mean, that the cells were generated through the destruction of an embryo. To the contrary, various methods are available and can be used to generate ES cells without destruction of an embryo, such as a human embryo. As an example, ES cells may be generated from single blastomeres derived from an embryo, in a manner similar to the extraction of blastomeres for pre-implantation genetic diagnosis (PGD). Examples of such cell lines include NED1, NED2, NED3, NED4, NED5, and NED7. An exemplary human embryonic stem cell line that may be used is MA09 cells. The isolation and preparation of MA09 cells was previously described in Klimanskaya, et al. (2006) "Human Embryonic Stem Cell lines Derived from Single Blastomeres." Nature 444: 481-485, and Chung et al. (2008) "Human Embryonic Stem Cell Lines Generated without Embryo Destruction, Cell Stem Cell, 2(2): 113-117. All of these lines were generated without embryo destruction.

Additional ES cells that may be used include human ES cells such as MA01, MA09, ACT-4, MA142, MA143, MA144, MA145, MA146, MA126 (NED1), MA127, (NED2), MA128, (NED3), MA129, (NED4), NED7, No. 3, H1, H7, H9, H14 and ACT30 ES cells.

Reference can also be made to the NIH Human Embryonic Stem Cell Registry. The human ES cells used in accordance with this disclosure may be derived and maintained in accordance with GMP standards which are known in the art.

As mentioned herein, ES cells have been derived from a variety of species including mice, multiple species of non-human primates, and humans, and embryo-derived stem cells have been generated from numerous additional species.

*Induced pluripotent stem (iPS) cells.* Induced pluripotent stem cells (iPS cells) are cells generated through the reprogramming of a somatic cell by expressing a combination of factors (herein referred to as reprogramming factors).

iPS cells can be generated using, as a starting point, virtually any somatic cell at any developmental stage. For example, the cell can be from an embryo, fetus, neonate, juvenile, or adult donor. In some instances, the cell is from a neonate, juvenile, or adult donor, or is obtained from the amniotic fluid, and thus does not require destruction of an embryo or fetus. Exemplary somatic cells that can be used include fibroblasts, such as dermal fibroblasts obtained by a skin sample or biopsy, synoviocytes from synovial tissue, foreskin cells, cheek cells, and lung fibroblasts. In certain embodiments, the somatic cell is not a fibroblast.

In certain embodiments, at least one, at least two, or at least three, or at least four reprogramming factors are expressed in a somatic cell to successfully reprogram the somatic cell. The reprogramming factors may be selected from Oct 4 (sometimes referred to as Oct 3/4), SOX2, c-Myc, Klf4, Nanog and Lin28. In some embodiments, the reprogramming factors include a combination of Oct 4 (sometimes referred to as Oct 3/4), SOX2, c-Myc and Klf4, or a combination of Oct 4, SOX2, Nanog and Lin28.

The reprogramming factors may be introduced into somatic cells using any of a variety of methods including integrative vectors, non-integrative vectors (e.g., an episomal plasmid, such as that described in Yu et al., Science. 2009 May 8;324(5928):797-801), chemical means, protein transduction, electroporation, microinjection, cationic amphiphiles, fusion with lipid bilayers, detergent permeabilization, etc., or a combination thereof. Additionally, expression of the reprogramming factors may be induced by contacting the somatic cells with at least one agent, such as a small organic molecule agents, that induce expression of reprogramming factors.

Expression of Oct3/4, SOX2, c myc and Klf4, using integrative viral vectors, has been shown to be sufficient to reprogram a mouse somatic cell. Expression of Oct3/4, SOX2, NANOG and Lin28, using integrative viral vectors, has been shown to be sufficient to reprogram a human somatic cell.

iPS cells may be obtained from a cell bank. iPS cells may be specifically generated using material from a particular patient or matched donor with the goal of generating tissue-matched RG cells or RG progenitor cells. iPS cells can be produced from cells that are not substantially immunogenic in an intended recipient, e.g., produced from autologous cells or from cells histocompatible to an intended recipient.

*Stimulus-triggered acquisition of pluripotency (STAP) cells.* Stimulus-triggered acquisition of pluripotency (STAP) cells are pluripotent stem cells produced by reprogramming somatic cells with sublethal stimuli such as low-pH exposure. The reprogramming does not require nuclear transfer into or genetic manipulation of the somatic cells. Reference can be made to Obokata et al., Nature, 505:676-680, 2014.

*Adult stem cells.* "Adult stem cell" refers to a multipotent cell isolated from adult tissue and can include bone marrow stem cells, cord blood stem cells and adipose stem cells and is of human origin.

Significantly, the methods of the invention do not employ an EB differentiation step (i.e., differentiation occurs from the pluripotent stem cells through to the retinal ganglion cells without the formation of EBs). As discussed herein, there are unexpected advantages to such a differentiation process.

"Embryoid bodies" refers to clumps or clusters of cells including pluripotent stem cells (e.g., iPSC or ESC), which may be formed by culturing pluripotent stem cells under non-attached conditions, e.g., on a low-adherent substrate or in a "hanging drop." In these cultures, pluripotent cells can form clumps or clusters of cells denominated as embryoid bodies. *See* Itskovitz-Eldor et al., Mol Med. 2000 Feb;6(2):88-95. Typically, embryoid bodies initially form as solid clumps or clusters of pluripotent cells, and over time some of the embryoid bodies come to include fluid filled cavities, the former being referred to in the literature as "simple" EBs and the latter as "cystic" embryoid bodies.

### Applications and Uses

### Screening Assays

The present disclosure describes methods for screening various agents that modulate the differentiation of an eye field progenitor cell towards the retinal ganglion lineage. It could also be used to discover therapeutic agents that support and/or rescue RG progenitor cells that may be generated in culture from eye field progenitor cells.

The present disclosure describes methods for screening various agents that modulate the differentiation of an RG progenitor cell. It could also be used to discover therapeutic agents that support and/or rescue mature RG cells that are generated in culture from RG progenitor cells.

For the purposes of this disclosure, an "agent" is intended to include a biological or chemical compound such as a simple or complex organic or inorganic molecule, a peptide, a protein (e.g. antibody), a polynucleotide (e.g. anti-sense, RNA interference (RNAi, shRNA, microRNA)) or a ribozyme. A vast array of compounds can be synthesized, for example polymers, such as polypeptides and polynucleotides, and synthetic organic compounds based on various core structures, and these are also included in the term "agent." In addition, various natural sources can provide compounds for screening, such as plant or animal extracts, and the like. It should be understood, although not always explicitly stated, that the agent is used alone or in combination with another agent, having the same or different biological activity as the agents identified by the inventive screen.

To practice the screening method *in vitro,* an isolated population of cells can be obtained as described herein. When the agent is a composition other than a DNA or RNA, such as a small molecule as described above, the agent can be directly added to the cells or added to culture medium. As is apparent to those skilled in the art, an "effective" amount must be added which can be empirically determined. When the agent is a polynucleotide, it can be directly added by use of a gene gun or electroporation. Alternatively, it can be inserted into the cell using a gene delivery vehicle or other method as described above. Positive and negative controls can be assayed to confirm the purported activity of the drug or other agent.

### Drug Screening

Also described herein is the development and use of assays for identifying drugs which are able to either protect ganglion from the effects of elevated IOP, glutamate toxicity or other environmental or genetic conditions which may cause loss of function or cell death of native retinal ganglion cells and their progenitors. Protection may be achieved by for example reducing the sensitivity of the ganglion to these various effects. In other embodiments, the assay can be used to identify agents which promote the proliferation of native retinal ganglion cells and/or their progenitors, and/or which may induce the differentiation/maturation of native retinal ganglion progenitors to mature retinal ganglion cells. Agents that may be tested in these assays include small molecules (e.g., organic compounds that have molecular weights of less than 10,000 atomic mass units (amu)), proteins and peptides, nucleic acids, etc. Agents to be tested can be produced, for example, by bacteria, yeast or other organisms (e.g. natural products), produced chemically (e.g. small molecules, including peptidomimetics), or produced recombinantly.

For example, the assay may evaluate the ability of a compound to protect retinal ganglion cells (such as may be derived by differentiation of pluripotent stem cells, eye field progenitors, or RG progenitor cells using the methods provided herein) in culture from glutamate induced toxicity. A variety of assay formats will suffice and, in light of the present disclosure, those not expressly described herein will nevertheless be comprehended by one of ordinary skill in the art. In many drug screening programs which test libraries of compounds and natural extracts, high throughput assays are desirable in order to maximize the number of compounds surveyed in a given period of time. Detection and quantification of protective activity of a test agent can be carried out, for example, by detecting the relative changes in markers of apoptosis - agents which are protective, should produce a decrease in the prevalence/level of expression of markers for apoptosis relative to control untreated cells under neurotoxic conditions such as elevated glutamate levels. The efficacy of the agent can be assessed by generating dose response curves from data obtained using various concentrations of the test compound. Control assays can also be performed to provide a baseline for comparison. In the control assay, the degree of apoptosis can be quantitated in the absence of the test compound. It will be understood that, in general, the order in which the reactants may be admixed can be varied, and can be admixed simultaneously.

### Neurosensory Retinal Structures

The RG progenitor cells, and optionally the RG cells differentiated therefrom, can be used to generate neurosensory retinal structures that include retinal pigment epithelial (RPE) cells, photoreceptor cells and the RG progenitor or RG cells themselves. For instance, the invention contemplates the generation of multilayer cellular structures comprised of RG cells or RG progenitor cells, RPE cells, and photoreceptor cells (or photoreceptor progenitor cells). These structures can be used for drug screening, as models for diseases, or in a pharmaceutical preparation. In the latter case, the pharmaceutical preparation can be an RPE-photoreceptor-RG graft, which may be disposed on a biocompatible solid support or matrix (preferably a bioresorbable matrix or support) that can be implanted like a "patch".

It is further contemplated that RG cells or RG progenitor cells can be generated along with photoreceptors from retinal neural progenitors (e.g., Chx10(+) cells), and these cells can be transplanted along with an RPE cell layer (e.g., a monolayer).

To further illustrate, the biocompatible support for the cells can be a biodegradable polyester film support for retinal progenitor cells. The biodegradable polyester can be any biodegradable polyester suitable for use as a substrate or scaffold for supporting the proliferation and differentiation of retinal progenitor cells. The polyester should be capable of forming a thin film, preferably a micro-textured film, and should be biodegradable if used for tissue or cell transplantation. Suitable biodegradable polyesters for use in the invention include polylactic acid (PLA), polylactides, polyhydroxyalkanoates, both homopolymers and co-polymers, such as polyhydoxybutyrate (PHB), polyhydroxybutyrate co-hydroxyvalerate (PHBV), polyhydroxybutyrate co-hydroxyhexanote (PHBHx), polyhydroxybutyrate co-hydroxyoctonoate (PHBO) and polyhydroxybutyrate co-hydroxyoctadecanoate (PHBOd), polycaprolactone (PCL), polyesteramide (PEA), aliphatic copolyesters, such as polybutylene succinate (PBS) and polybutylene succinate/adipate (PBSA), aromatic copolyesters. Both high and low molecular weight polyesters, substituted and unsubstituted polyester, block, branched or random, and polyester mixtures and blends can be used. Preferably the biodegradable polyester is polycaprolactone (PCL).

In certain embodiments, the biocompatible support is a poly(p-xylylene) polymer, such as parylene N, parylene D, parylene-C, parylene AF-4, parylene SF, parylene HT, parylene VT-4 and Parylene CF, and most preferably parylene-C.

The polymeric support can typically be formed into a thin film using known techniques. The film thickness is advantageously from about 1 micron to about 50 microns, and preferably about 5 microns in thickness. The surface of the film can be smooth, or the film surface can be partially or completely micro-textured. Suitable surface textures include micro-grooves or micro-posts, for instance. The film can be cut and shaped to form a suitable shape for implantation.

The RPE, photoreceptor cells or photoreceptor progenitor cells, and RG progenitor cells and/or RG cells can be plated directly, together or sequentially (e.g., photoreceptor cells or photoreceptor progenitor cells after an RPE layer is formed), onto the film to form a biocompatible scaffold. Alternatively, the polymer film can be coated with a suitable coating material such as poly-D-lysine, poly-L-lysine, fibronectin, laminin, collagen I, collagen IV, vitronectin and Matrigel™. The cells can be plated to any desired density, but a single layer of RPE cells (an RPE monolayer) is preferred.

### Therapeutic Uses

The present invention provides a composition in accordance with the claims for use in a method of treatment of a mammalian patient, comprising administering the composition to said patient, or administering the composition after thawing to said patient. In an exemplary embodiment, the cells may be transplanted into a rat in need thereof, e.g., an intraocular hypertension rat, or other animal model of disease, and the resulting effect on visual function may be detected by the Optomotor response test, ERG (electroretinography), luminance threshold recording and/or the visual center blood flow assay.

"Signs" of disease, as used herein, refers broadly to any abnormality indicative of disease, discoverable on examination of the patient; an objective indication of disease, in contrast to a symptom, which is a subjective indication of disease.

"Symptoms" of disease as used herein, refers broadly to any morbid phenomenon or departure from the normal in structure, function, or sensation, experienced by the patient and indicative of disease.

"Therapy," "therapeutic," "treating," "treat" or "treatment", as used herein, refers broadly to treating a disease, arresting or reducing the development of the disease or its clinical symptoms, and/or relieving the disease, causing regression of the disease or its clinical symptoms. Therapy encompasses prophylaxis, prevention, treatment, cure, remedy, reduction, alleviation, and/or providing relief from a disease, signs, and/or symptoms of a disease. Therapy encompasses an alleviation of signs and/or symptoms in patients with ongoing disease signs and/or symptoms. Therapy also encompasses "prophylaxis" and "prevention". Prophylaxis includes preventing disease occurring subsequent to treatment of a disease in a patient or reducing the incidence or severity of the disease in a patient. The term "reduced", for purpose of therapy, refers broadly to the clinical significant reduction in signs and/or symptoms. Therapy includes treating relapses or recurrent signs and/or symptoms. Therapy encompasses precluding the appearance of signs and/or symptoms anytime as well as reducing existing signs and/or symptoms and eliminating existing signs and/or symptoms. Therapy includes treating chronic disease ("maintenance") and acute disease. For example, treatment includes treating or preventing relapses or the recurrence of signs and/or symptoms.

In some embodiments, treatment may be indicated by stable or improved scoptic threshold response, stable or improved optokinetic reflex (OKR), stable or improved vision evoked potential (VEP), and/or stable or improved visual acuity (e.g., as measured by ERG). Stable or improved levels are relative to levels pre-treatment or relative to non-treated controls such as contralateral eyes. It is to be understood that in some instances one or more of these functions or responses will deteriorate with time in the absence of treatment, and that the transplanted cells of the invention may impede, reduce or limit that deterioration. Thus in some instances the function or response remains stable relative to control while in other instances it improves, and both situations are considered to be beneficial to the subject and thus to be regarded as treatment.

In these and other embodiments, the subject may experience an underlying benefit to its retinal ganglion cell layer. For example, the native RG cell layer may experience improved survival, or reduced cell loss, compared to a control. In still other embodiments, the subject may experience an increased formation of axons in the optic nerve.

This invention also provides compositions that may be for use in methods for replacing or repairing RG cells in a patient in need of this treatment comprising administering a pharmaceutical preparation including the RG progenitor cells of the present invention, or RG cells derived therefrom, or a combination thereof, to a patient. As described herein, the pharmaceutical preparation can be a suspension of cells or cells which are formed into transplantable tissue *in vitro.* In many instances, the cells will be administered by intraocular injection (including vitreal injection and subretinal injection). Thus the cells may be administered intraocularly into the sub-retinal space of a diseased or degenerated retina. However, as the RG progenitor cells also have a neuroprotective effect, the cells can be administered locally but outside of the retina (such as in the vitreous) or by depot or systemic delivery to other parts of the body.

The pharmaceutical preparations of the present invention can be used in a wide range of diseases and disorders that result in visual system deterioration, including retinal degeneration-related disease. Such diseases and disorders may be caused by aging, such that there appears to be an absence of an injury or disease that is identifiable as a substantial source of the deterioration. Skilled artisans will understand the established methods for diagnosing such disease states, and/or inspecting for known signs of such injuries. In addition, the literature is replete with information on age-related decline or deterioration in aspects of the visual systems of animals. The term "retinal degeneration-related disease" is intended to refer to any disease resulting from innate or postnatal retinal degeneration or abnormalities. Examples of retinal degeneration-related diseases include retinal dysplasia, aged macular degeneration, diabetic retinopathy, retinitis pigmentosa, congenital retinal dystrophy, Leber congenital amaurosis, retinal detachment, glaucoma, optic neuropathy, and trauma.

Additionally or alternatively, the deterioration of the visual system components, such as the neurosensory retina can be caused by injury, for example trauma to the visual system itself (e.g., an eye), to the head or brain, or the body more generally. Certain such injuries are known to be age-related injuries, i.e., their likelihood, or frequency increases with age. Examples of such injuries include retinal tears, macular holes, epi-retinal membrane, and retinal detachments, each of which might occur in an animal of any age, but which are more likely to occur, or occur with greater frequency in aging animals, including otherwise healthy aging animals. In other instances, deterioration of the visual system components may occur due to ischemic and/or reperfusion injury.

The deterioration of the visual system or components thereof also can be caused by disease. Included among the diseases are various age-related diseases that impact the visual system. Such diseases occur with greater likelihood and/or frequency in older animals than in the young. Examples of diseases which may affect the visual system, including for example the neurosensory retinal layers, and cause deterioration thereof are various forms of retinitis, optic neuritis, macular degeneration, proliferative or nonproliferative diabetic retinopathy, diabetic macular edema, progressive retinal atrophy, progressive retinal degeneration, sudden acquired retinal degeneration, immune-mediated retinopathy, retinal dysplasia, chorioretinitis, retinal ischemia, retinal hemorrhage (preretinal, intraretinal and/or subretinal), hypertensive retinopathy, retinal inflammation, retinal edema, retinoblastoma, or retinitis pigmentosa.

Some of the foregoing diseases tend to be specific to certain animals such as companion animals, e.g., dogs and/or cats. Some of the diseases are listed generically, i.e., there may be many types of retinitis, or retinal hemorrhage; thus some of the diseases are not caused by one specific etiologic agent, but are more descriptive of the type of disease or the result. Many of the diseases that can cause decline or deterioration of one or more components of the visual system can have both primary and secondary or more remote effects on an animal's visual system.

Advantageously, the pharmaceutical preparations of the present invention may be used to treat or ameliorate a number of conditions including ischemia reperfusion injury, retinal vascular disease, optic nerve injury (whether autoimmune, trauma, and/or viral-based), glaucoma (regardless of type), diabetic retinopathy (whether hypoxic and angiogenic based), inherited ganglion degeneration, and hereditary optic neuropathy (such as Leber's hereditary optic neuropathy (LHON) or Leber optic atrophy).

In one aspect, the cells can treat or alleviate the symptoms of any of the foregoing conditions in a patient in need of the treatment. The cells can be autologous or allogeneic to the patient. In a further aspect, the cells of the invention can be administered in combination with other treatments.

The RG progenitor cells of the present invention find use in the treatment of degenerative diseases. The cells are administered in a manner that permits them to graft or migrate to the intended ocular site, such as the ganglion cell layer of the retina, and reconstitute or regenerate the functionally deficient area.

Genetically engineered RG progenitor cells or RG cells can also be used to target gene products to sites of degeneration, and can, for example, be engineered with one or more genes that encode secreted biologically active proteins, peptides, or nucleic acids (such as RNA interference molecules or aptamers), or the like. These gene products can include survival-promoting factors to rescue native degenerating neurons, factors that can act in an autocrine manner to promote survival and differentiation of grafted cells into site-specific neurons or to deliver neurotransmitter(s) to permit functional recovery. For example, the one or more biologically active molecule(s) can include anti-angiogenic antibodies and molecules, anti-angiogenic antibody scaffolds, soluble receptors, agents targeting and inhibiting or modulating immunologic pathway molecules, growth factor inhibitors, growth factors, neurotrophic factors, angiogenic factors, neurotransmitters, hormones, enzymes, anti-inflammatory factors, or other therapeutic proteins. In various embodiments, such molecules can include C3a inhibitors, C3b inhibitors, other agents targeting and inhibiting or modulating immunologic pathway molecules, brain derived neurotrophic factor (BDNF), NT-4, ciliary neurotrophic factor (CNTF), Axokine, basic fibroblast growth factor (bFGF), insulin-like growth factor I (IGF I), insulin-like growth factor II (IGF II), acid fibroblast growth factor (aFGF), fibroblast growth factor (aFGF), epidermal growth factor (EGF), transforming growth factor-alpha (TGF-alpha), transforming growth factor-beta (TGF-beta), nerve growth factor (NGF), platelet derived growth factor (PDGF), glia-derived neurotrophic factor (GDNF), Midkine, tryophotin, activin, thyrotropin releasing hormone, interleukins, bone morphogenic protein, macrophage inflammatory proteins, heparin sulfate, amphiregulin, retinoic acid, tumor necrosis factor alpha, fibroblast growth factor receptor, epidermal growth factor receptor (EGFR), PEDF, LEDGF, NTN, Neublastin, neurotrophins, lymphokines, VEGF inhibitors, PDGF inhibitors, placental growth factor (PIGF) inhibitors, Tie2, CD55, C59, a bispecific molecule that simultaneously binds VEGF and PDGF, and other agents expected to have therapeutically useful effects on potential target tissues. In certain preferred embodiments, the recombinantly engineered RG progenitor cells or RG cells are engineered to express one or more recombinant growth factors and neurotrophins such as FGF2, NGF, ciliary neurotrophic factor (CNTF), and brain derived neurotrophic factor (BDNF), which factors have been shown to significantly slow the process of cell death in models of retinal degeneration. Therapy using RG progenitor and/or RG cells engineered to synthesize a growth factor or a combination of growth factors can not only ensure sustained delivery of neuroprotectants, but may also reconstruct damaged retina.

In certain embodiments, the gene construct used to engineer the RG progenitor or RG cell can include transcriptional and/or translational control elements (such as enhancers or promoters) which are sensitive to conditions which would promote ganglion cell death and/or glaucoma, such as elevated intraocular pressure (IOP), elevated levels of glutamate, etc. An exemplary promoter sensitive to elevated IOP is the promoter from the matrix Gla protein (MGP) gene (see, for example Gonzalez et al., Investigative Ophthalmology & Visual Science, May 2004, 45(5): 1389). Selective targeting has also been achieved using the 5' promoter region of the chitinase 3-like 1 ("Ch3L1") gene, with expression specifically directed to the outermost anterior and posterior regions of the TM (Liton et al., Invest Ophthalmol Vis Sci. 2005, 46:183). Further, numerous gene profiling studies of the trabecular meshwork have been published, providing additional alternative configurations for trabecular meshwork cell-selective promoters (Gonzalez et al., Invest Ophthalmol Vis Sci. 2000, 41:3678-3693; Wirtz et al., Invest Ophthalmol Vis Sci. 2002, 43:3698; Tomarev et al., Invest Ophthalmol Vis Sci. 2003, 44:2588-2596; Liton et al., Invest Ophthalmol Vis Sci. 2005, 46:183; Liton et al., Mol Vis. 2006, 12:774; Fan et al., Invest Ophthalmol Vis Sci. 2008, 49:1886; Fuchshofer et al., Exp Eye Res. 2009, 88:1020; Paylakhi et al., Mol Vis. 2012, 18:241; and Liu et al., Invest Ophthalmol Vis Sci. 2013, 54:6382).

In the methods of the invention, cells to be transplanted are transferred to a recipient in any physiologically acceptable excipient comprising an isotonic excipient prepared under sufficiently sterile conditions for human administration. For general principles in medicinal formulation, the reader is referred to Cell Therapy: Stem Cell Transplantation, Gene Therapy, and Cellular Immunotherapy, by G. Morstyn & W. Sheridan eds, Cambridge University Press, 1996. Choice of the cellular excipient and any accompanying elements of the composition will be adapted in accordance with the route and device used for administration. The cells may be introduced by injection, catheter, or the like. The cells may be frozen at liquid nitrogen temperatures and stored for long periods of time, being capable of use on thawing. If frozen, the cells may be stored in a 10% DMSO, 50% FCS, 40% RPMI 1640 medium. In a preferred embodiment, the cells will be stored in an animal-free cryopreservation buffer such as Cryostor CS10.

The pharmaceutical preparations of the invention are optionally packaged in a suitable container with written instructions for a desired purpose. Such formulations may comprise a cocktail of retinal differentiation and/or trophic factors, in a form suitable for combining with RG progenitor or RG cells. Such a composition may further comprise suitable buffers and/or excipients appropriate for transfer into an animal. Such compositions may further comprise the cells to be engrafted.

### Pharmaceutical Preparations

The RG progenitor cells or RG cells may be formulated with a pharmaceutically acceptable carrier. For example, RG progenitor cells or RG cells may be administered alone or as a component of a pharmaceutical formulation. The subject compounds may be formulated for administration in any convenient way for use in medicine. Pharmaceutical preparations suitable for administration may comprise the RG progenitor cells or RG cells, in combination with one or more pharmaceutically acceptable sterile isotonic aqueous or non-aqueous solutions (e.g., balanced salt solution (BSS)), dispersions, suspensions or emulsions, or sterile powders which may be reconstituted into sterile injectable solutions or dispersions just prior to use, which may contain antioxidants, buffers, bacteriostats, solutes or suspending or thickening agents. Exemplary pharmaceutical preparations comprise the RG progenitor cells or RG cells in combination with ALCON® BSS PLUS® (a balanced salt solution containing, in each ml, sodium chloride 7.14 mg, potassium chloride 0.38 mg, calcium chloride dihydrate 0.154 mg, magnesium chloride hexahydrate 0.2 mg, dibasic sodium phosphate 0.42 mg, sodium bicarbonate 2.1 mg, dextrose 0.92 mg, glutathione disulfide (oxidized glutathione) 0.184 mg, hydrochloric acid and/or sodium hydroxide (to adjust pH to approximately 7.4) in water).

When administered, the pharmaceutical preparations for use in this disclosure may be in a pyrogen-free, physiologically acceptable form.

The preparation comprising RG progenitor cells or RG cells may be transplanted in a suspension, gel, colloid, slurry, or mixture. Further, the preparation may desirably be encapsulated or injected in a viscous form into the vitreous humor for delivery to the site of retinal or choroidal damage. Also, at the time of injection, cryopreserved RG progenitor cells or RG cells may be resuspended with balanced salt solution to achieve the desired osmolality and concentration for administration by subretinal injection. The preparation may be administered to an area of the pericentral macula that was not completely lost to disease, which may promote attachment and/or survival of the administered cells. In some instances, including for example the glaucoma animal model, the RG progenitors and/or RG cells are administered intravitreously.

The RG progenitors cells and/or RG cells may be frozen (cryopreserved) as described herein. Upon thawing, the viability of such cells may be at least 20%, at least 30%, at least 40%, at least 50%, at least 60%, at least 70%, at least 80%, at least 90% at least 95% or about 100% (e.g., at least 20%, at least 30%, at least 40%, at least 50%, at least 60%, at least 70%, at least 80%, at least 90% at least 95% or about 100% of the cells harvested after thawing are viable or at least 20%, at least 30%, at least 40%, at least 50%, at least 60%, at least 70%, at least 80%, at least 90% at least 95% or about 100% of the cell number initially frozen are harvested in a viable state after thawing).

The RG progenitor cells or RG cells may be formulated and delivered in a pharmaceutically acceptable ophthalmic vehicle or formulation suitable for intraocular injection. In some instances, the preparation is designed such that it is maintained in contact with the ocular surface for a sufficient time period to allow the cells to penetrate the affected regions of the eye, as for example, the anterior chamber, posterior chamber, vitreous body, aqueous humor, vitreous humor, cornea, iris/ciliary, lens, choroid, retina, sclera, suprachoridal space, conjunctiva, subconjunctival space, episcleral space, intracorneal space, epicorneal space, pars plana, surgically-induced avascular regions, or the macula.

The RG progenitor cells or RG cells may be contained in a sheet of cells. For example, a sheet of cells comprising RG progenitor cells or RG cells may be prepared by culturing RG progenitor cells or RG cells on a substrate from which an intact sheet of cells can be released, e.g., a thermoresponsive polymer such as a thermoresponsive poly(N-isopropylacrylamide) (PNIPAAm)-grafted surface, upon which cells adhere and proliferate at the culture temperature, and then upon a temperature shift, the surface characteristics are altered causing release of the cultured sheet of cells (e.g., by cooling to below the lower critical solution temperature (LCST). See, for example, da Silva et al., Trends Biotechnol. 2007 Dec;25(12):577-83; Hsiue et al., Transplantation 2006 Feb 15;81(3):473-6; Ide, T. et al. (2006); Biomaterials 27, 607-614, Sumide, T. et al. (2005), FASEB J. 20, 392-394; Nishida, K. et al. (2004), Transplantation 77, 379-385; and Nishida, K. et al. (2004), N. Engl. J. Med. 351, 1187-1196). The sheet of cells may be adherent to a substrate suitable for transplantation, such as a substrate that may dissolve *in vivo* when the sheet is transplanted into a host organism, e.g., prepared by culturing the cells on a substrate suitable for transplantation, or releasing the cells from another substrate (such as a thermoresponsive polymer) onto a substrate suitable for transplantation. An exemplary substrate suitable for transplantation may comprise gelatin (see Hsiue et al., supra). Alternative substrates suitable for transplantation include fibrin-based matrixes and others. The sheet of cells may be used in the manufacture of a medicament for the prevention or treatment of a disease of retinal degeneration.

The sheet of cells may be introduced into an eye in need thereof in conjunction with surgery or other invasive therapy including for example subfoveal membranectomy with transplantation of the sheet of RG progenitor cells or RG cells. Accordingly, the sheet of cells may be used in the manufacture of a medicament for transplantation in conjunction with subfoveal membranectomy.

The volume of preparation administered according to the methods described herein may be dependent on factors such as the mode of administration, number of RG progenitor cells or RG cells, age and weight of the patient, and type and severity of the disease being treated. When administering the formulation locally into the eye such as for example by intravitreal injection, the formulation should be sufficiently concentrated so that minimal volumes may be delivered. For example, if administered by injection, the volume of a pharmaceutical preparations of RG progenitor cells or RG cells may be about 1, 1.5, 2, 2.5, 3, 4, or 5 ml, or about 1-2 ml. For example, if administered by injection, the volume of a pharmaceutical preparation of RG progenitor cells or RG cells may be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 100, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, or 200 µL (microliters). For example, the volume of a preparation of the disclosure may be about 10-50, 20-50, 25-50, or 1-200 µL. The volume of a preparation of the disclosure may be about 10, 20, 30, 40, 50, 100, 110, 120, 130, 140, 150, 160, 170, 180, 190, or 200 µL, or higher.

Concentrations for injections may be at any level that is effective and non-toxic, depending upon the factors described herein. The pharmaceutical preparations of RG progenitor cells or RG cells for treatment of a patient may be formulated at doses of at least about 10³ cells/ml, including at least about 10³, 10⁴, 10⁵, 10⁶, 107, 10⁸, 10⁹, or 10¹⁰ RG progenitor cells or RG cells /ml. The pharmaceutical preparation may comprise at least about 1x10³, 2x10³, 3x10³, 4x10³, 5x10³, 6x10³, 7x10³, 8x10³, 9x10³, 1x10⁴, 2x10⁴, 3x10⁴, 4x10⁴, 5x10⁴, 6x10⁴, 7x10⁴, 8x10⁴, or 9x10⁴ RG progenitor cells or RG cells per µL. The preparation may comprise 2000 RG progenitor cells or RG cells per µL, including 100,000 RG progenitor cells or RG cells per 50 µL or 180,000 RG progenitor cells or RG cells per 90 µL.

The pharmaceutical preparations of RG progenitor cells or RG cells may comprise at least about 1,000; 2,000; 3,000; 4,000; 5,000; 6,000; 7,000; 8,000; or 9,000 RG progenitor cells or RG cells. The pharmaceutical preparations of RG progenitor cells or RG cells may comprise at least about 1x10⁴, 2x10⁴, 3x10⁴, 4x10⁴, 5x10⁴, 6x104, 7x10⁴, 8x10⁴, 9x10⁴, 1x10⁵, 2x10⁵, 3x10⁵, 4x10⁵, 5x10⁵, 6x10⁵, 7x10⁵, 8x10⁵, 9x10⁵, 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, or 9x10¹⁰ RG progenitor cells or RG cells. The pharmaceutical preparations of RG progenitor cells or RG cells may comprise at least about 1x10²-1x10³, 1x10²-1x10⁴, 1x10⁴-1x10⁵, or 1x10³-1x10⁶ RG progenitor cells or RG cells. The pharmaceutical preparations of RG progenitor cells or RG cells may comprise at least about 10,000, 20,000, 25,000, 50,000, 75,000, 100,000, 125,000, 150,000, 175,000, 180,000, 185,000, 190,000, or 200,000 RG progenitor cells or RG cells. For example, the pharmaceutical preparation of RG progenitor cells or RG cells may comprise at least about 20,000-200,000 RG progenitor cells or RG cells in a volume at least about 50-200 µL. Further, the pharmaceutical preparation of RG progenitor cells or RG cells may comprise about 50,000 RG progenitor cells or RG cells in a volume of 150 µL, about 200,000 RG progenitor cells or RG cells in a volume of 150 µL, or at least about 180,000 RG progenitor cells or RG cells in a volume at least about 150 µL.

In the aforesaid pharmaceutical preparations and compositions, the number of RG progenitor cells or RG cells or concentration of RG progenitor cells or RG cells may be determined by counting viable cells and excluding non-viable cells. For example, non-viable RG progenitor cells or RG cells may be detected by failure to exclude a vital dye (such as Trypan Blue), or using a functional assay (such as the ability to adhere to a culture substrate, etc.). Additionally, the number of RG progenitor cells or RG cells or concentration of RG progenitor cells or RG cells may be determined by counting cells that express one or more RG progenitor cell or RG cell markers and/or excluding cells that express one or more markers indicative of a cell type other than RG progenitor cells or RG cells.

The method of treating retinal degeneration may further comprise administration of an immunosuppressant. Immunosuppressants that may be used include anti-lymphocyte globulin (ALG) polyclonal antibody, anti-thymocyte globulin (ATG) polyclonal antibody, azathioprine, BASILIXIMAB® (anti-IL-2Rα receptor antibody), cyclosporin (cyclosporin A), DACLIZUMAB® (anti-IL-2Rα receptor antibody), everolimus, mycophenolic acid, RITUXIMAB® (anti-CD20 antibody), sirolimus, and tacrolimus. The immunosuppressants may be dosed at least about 1, 2, 4, 5, 6, 7, 8, 9, or 10 mg/kg. When immunosuppressants are used, they may be administered systemically or locally, and they may be administered prior to, concomitantly with, or following administration of the RG progenitor cells or RG cells. Immunosuppressive therapy may continue for weeks, months, years, or indefinitely following administration of RG progenitor cells or RG cells. For example, the patient may be administered 5 mg/kg cyclosporin for 6 weeks following administration of the RG progenitor cells or RG cells.

The method of treatment of retinal degeneration may comprise the administration of a single dose of RG progenitor cells or RG cells. Alternatively, the methods of treatment described herein may comprise a course of therapy where RG progenitor cells or RG cells are administered multiple times over some period. Exemplary courses of treatment may comprise weekly, biweekly, monthly, quarterly, biannually, or yearly treatments. Alternatively, treatment may proceed in phases whereby multiple doses are administered initially (e.g., daily doses for the first week), and subsequently fewer and less frequent doses are needed.

If administered by injection, including intraocular injection, the RG progenitor cells or RG cells may be delivered one or more times periodically throughout the life of a patient. For example, the RG progenitor cells or RG cells may be delivered once per year, once every 6-12 months, once every 3-6 months, once every 1-3 months, or once every 1-4 weeks. Alternatively, more frequent administration may be desirable for certain conditions or disorders. If administered by an implant or device, the RG progenitor cells or RG cells may be administered one time, or one or more times periodically throughout the lifetime of the patient, as necessary for the particular patient and disorder or condition being treated. Similarly contemplated is a therapeutic regimen that changes over time. For example, more frequent treatment may be needed at the outset (e.g., daily or weekly treatment). Over time, as the patient's condition improves, less frequent treatment or even no further treatment may be needed.

The methods described herein may further comprise the step of monitoring the efficacy of treatment or prevention by measuring electroretinogram responses, optomotor acuity threshold, or luminance threshold in the subject. The method may also comprise monitoring the efficacy of treatment or prevention by monitoring immunogenicity of the cells or migration or intregration or survival of the cells in the eye.

The RG progenitor cells or RG cells may be used in the manufacture of a medicament to treat retinal degeneration. The disclosure also encompasses the use of the preparation comprising RG progenitor cells or RG cells in the treatment of blindness. For example, the preparations comprising human RG progenitor cells or RG cells may be used to treat retinal degeneration associated with a number of vision-altering ailments that result in retinal damage and blindness, such as ischemia reperfusion injury, retinal vascular disease, optic nerve injury (whether autoimmune, trauma, and/or viral-based), glaucoma (regardless of type), diabetic retinopathy (whether hypoxic and angiogenic-based), and hereditary optic neuropathy (such as Leber's hereditary optic neuropathy (LHON) or Leber optic atrophy). In some embodiments, the preparations comprising human RG progenitor cells or RG cells may be used alone or together with other therapeutics (such as other cellular therapies such as RPE cell and/or photoreceptor cell populations) to treat macular degeneration (including age related macular degeneration, e.g., wet age related macular degeneration and dry age related macular degeneration), retinitis pigmentosa, and Stargardt's Disease (fundus flavimaculatus), night blindness and color blindness. The preparation may comprise at least about 5,000-500,000 RG progenitor cells or RG cells (e.g., 100,000 RG progenitor cells or RG cells).

Either or both RG progenitor cells and RG cells may be used. Human cells may be used in human patients, as well as in animal models or animal patients. For example, the human cells may be tested in mouse, rat, cat, dog, or non-human primate models of retinal degeneration. Additionally, the human cells may be used therapeutically to treat animals in need thereof, such as in veterinary medicine.

As defined here, singular forms are provided for illustrative purposes, but may also apply to plural versions of the phrase.

The following are examples to illustrate the invention.

### EXAMPLES

### Example 1. RG progenitor cell and RG cell production from pluripotent stem cells

1. Human embryonic stem cells are cultured under the feeder free conditions in mTESR1 media (Stem Cell Technology) on Matrigel™ (BD Biosciences). Upon 80-90% confluence, the cells need to be passaged or frozen. Passaging of stem cells is performed using enzymatic (e.g., dispase) or non-enzymatic (e.g., PBS-based cell dissociation buffer, Invitrogen) techniques.
2. Direct differentiation methods are used for generation of retinal neurons/progenitors. There is no embryonic body step.
3. Day 0: Cell differentiation is induced at 15-20% confluence. Change media to retinal induction (RI) media: DMEM/F12 supplied with 4.5 mg/ml D-glucose, 100 unit/ml of penicillin, 100 µg/ml of streptomycin, 1% N2 supplement (Invitrogen)(0.1-2%), 0.2% B27 supplement (0.05-2%), IX MEM Non-essential amino acids solution, 50 ng/ml Noggin (10-500 ng/ml), and 20 µg/ml human insulin(5-50 µg/ml).
4. Day 1-Day 4: Complete media change every day.
5. Day 5: Cell cultures become 80-90% confluent on Day 5. Media is changed to neural differentiation (ND) media: Neurobasal™ Medium (Life Technologies) supplied with 4.5 mg/ml D-glucose, 100 unit/ml of penicillin, 100 µg/ml of streptomycin, IX GlutaMAX™, 1% N2 supplement (Invitrogen) (0.1-2%), 2% B27 supplement (0.5-2%), IX MEM Non-essential amino acids solution, and 50 ng/ml Noggin (10-500 µg/ml).
6. Day 6-Day 13: Cells are maintained in the ND media. Change medium every 2 days. Cell colonies continue to grow in the ND media. The edge cells become flat and large, while the central cells are smaller and form compact cell clusters. At Days 10-11, cells start expressing PAX6 followed by onset of Rxl expression on Day 12 (FIG. 1A). RT-PCR showed loss of Oct4 and Nanog expression (FIG. 1B). On Day 13, over 90% cells co-express Pax6 and Rx1 (FIG. 2A) as quantified by flow cytometry. Over 99% of cells express Otx2, Nestin and Sox2 (FIG. 2B). These results indicate that they become eye field progenitor cells.
7. Day 14-Day 19: At Day 15, change medium to Ganglion cell (GC) medium: Neural basal media supplied with 4.5 mg/ml D-glucose, 100 unit/ml of penicillin, 100 µg/ml of streptomycin, IX GlutaMAX™, 1% N2 supplement (0.1-2%), 2% B27 supplement (formula number 080085-SA)(0.5-2%), 5 µM forskolin (1-20µM), 10ng/ml BDNF (1-200ng/ml), and 10ng/ml CNTF (1-200ng/ml).
8. Day 20-Day 35: At Day 20, cells are lifted off from growth surface and mechanically fragmented into clusters in GC medium. Transfer cell clusters to 100 mm ultra-low attachment culture dishes. Cell clusters round up and form individual spheres in the suspension culture. Change medium every 3 days. At Day 35, real-time RT-PCR showed a strong up-regulation of the expression of Math5, Brn3a, Brn3b and Isl1 (FIG. 3A). Flow cytometry showed >99% cells expressed transcription factors Math5 and Brn3a. About 94% Math5 positive cells stained negative for PAX6 (FIG. 3B).
9. Day 36-Day 60: Maintain neural sphere cultures in GC medium and change medium every 3-4 days. At day 60, real-time RT-PCR showed continued expression of Brn3a. The expression levels of Math5 and Brn3b were greatly down-regulated (FIG. 4). Flow cytometry showed 95.3% of cells co-expressed Brn3a and Neurofilament.
10. Differentiation of RG progenitors cells into RG cells:
   a. Cells are dissociated into single cells using Accutase® cell dissociation buffer. Cells are plated on poly-D-lysine/laminin-coated plate at the density of 10-40/cm² in RGC differentiation medium: Neural basal media supplied with 4.5 mg/ml D-glucose, 100 unit/ml of penicillin, 100 µg/ml of streptomycin, IX GlutaMAX™, 2% B27 supplement (formula number 080085-SA, same as GC medium), 5 µM forskolin, 10 ng/ml BDNF, 10ng/ml CNTF, 100ng/ml cAMP, 1-50 µM DAPT, 2 µM retinoic acid and 20 µg/ml human insulin. Change half media every 2 days.
   b. After 2 weeks, about 72% of cells stained positive for Thy1 (FIG. 5). Cells had dendrites and axons (FIG. 6A). Immunostaining showed expression of Brn3a, Neurofilament and Tuj 1 (FIG. 6B).

### Example 2. RG Progenitor Cells in an Experimental Glaucoma Model (using laser photocoagulation)

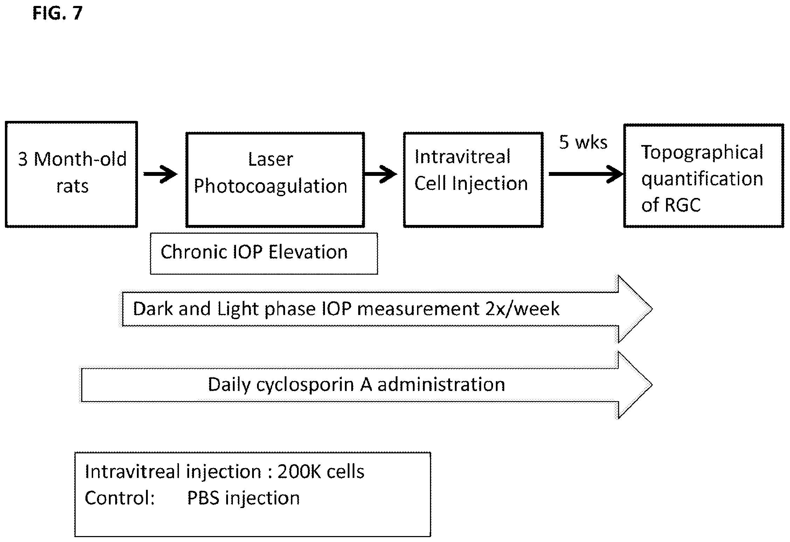

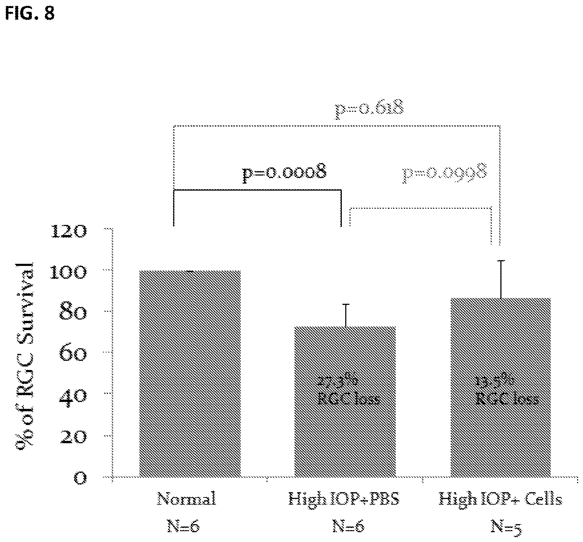

The effect of the RG progenitor cells *in vivo* was studied using an experimental glaucoma animal model, as outlined in FIG. 7. In this model system, chronic intraocular pressure (IOP) elevation was induced by trabecular laser photocoagulation in 3 month-old male Brown Norway rats. 2x10⁵ RG (early) progenitor cells suspended in 5 µl of PBS were injected into the vitreous cavity on the next day. PBS was injected into control eye balls. All rats received dark and light phase IOP measurement by TonoLab 2 times per week (from 2 weeks before the treatment to 5 weeks after the treatment). Cyclosporin A was administered in water from 1 week before treatment to 5 weeks after treatment. At 5 weeks after treatment, topographical quantification of retinal ganglion (RG) cells was performed using Rbpms immunohistochemistry. There was an average cell loss of 27.3% RG cells in control eyes whereas in the cell treatment group there was significantly less RG cell death averaging 13.5% (FIG. 8). This result suggests a rescue of RG cell death by RG progenitor cell treatment in the glaucoma rat model.

### Example 3: Cryopreservation of human RG progenitor cells and RG cells

Early RG progenitor cells (step 8 in Example 1) and late RG progenitor cells (step 9 in Example 2) can be frozen as neurospheres in an animal-free cryopreservation buffer, such as Cryostor CS10, or another cyropreservation buffer such as 90% FBS and 10% DMSO. RG cells may be cryopreserved in these buffers as well.

### Example 4: Transplantation of RGCPs derived from hESCs and/or iPSCs in an experimental glaucoma model (microbead (MB) injection)

### Generation of RGCPs from hESCs and iPSCs

The hESC line GFP-H1 (NIH registered under WA01, expressing GFP for easy identification *in vivo*) and iPSC line HA1 (generated by mRNA with no genomic integration³¹) are maintained as previously reported.³¹ In a process routinely and continuously performed as described herein, these cells are first differentiated into EFPs, followed by generation of RGCPs at different developmental stages by replating EFPs onto Matrigel and culturing under conditions as described herein. During the differentiation process, the expression of EFP genes (Pax6, Rx1, Nestin and Sox2) is monitored. Before *in vivo* transplantation, late-stage RGCPs (Neurofilament+ Brn3a+ Math5-) are collected and dissociated into single cell suspensions using accutase.

### RGCP transplantation into glaucoma mice

Adult C57BL/6J mice received anterior chamber injections of either PBS (as controls) or microbeads as previously described to induce ocular hypertension (glaucoma).²⁹ PBS injected controls which have a normal IOP were added to the group of recipient mice to address if the survival of engrafted RGCPs is affected by the glaucoma disease process.

At 7 days after PBS or microbead injection, GFP-hESC- or HA1-iPSC-derived RGCPs (100,000 cells/µl) or vehicle control solution were injected intravitreally into both normal IOP (PBS-injected) and glaucomatous (microbead-injected) mice. This time point was selected for transplantation to better align with clinical presentation of patients manifesting disease symptoms. In agreement with other reports,⁹ in preliminary studies the grafted cells were observed to survive up to 8 weeks post transplantation in the absence of an immune suppressor in the mouse eye which is considered an immune privileged site (FIGs. 13, 14, and 16).

pSTR, which monitors electrical activities of RGCs in the retina, was measured at 4 and 8 week time points, with a first measurement taken from 1-2 days before microbead injection to obtain baseline values. Control and glaucomatous mice were analyzed using the pSTR test at 4 and 8 weeks.

Another test that was used to assess the therapeutic efficacy of the transplanted RGCPs is the optokinetic reflex (OKR). OKR measures visual acuity and contrast sensitivity through tracking a drifting stripe pattern with eye and head movement. Glaucomatous mice exhibit drastically decreasing visual contrast sensitivity without compromised visual acuity in an OKR assay. These observations are in agreement with that seen in human patients.^{37,38} Baseline OKR were recorded at 1-2 days before microbead injection and OKR was assessed at 8 weeks after PBS or microbead injection for continuing evaluation of visual function and retinal morphology changes.

To test if immune suppression is needed to maintain grafted cell survival, mice subjected to treatment with the immune suppressor cyclosporine-A are studied. This group of mice receives daily treatment of cyclosporine-A in drinking water starting 1 day before transplantation until sacrifice.

Another test to be used to assess the therapeutic efficacy of the transplanted RGCPs is vision evoked potential (VEP). VEP measures a gross electrical signal generated by the cortex in response to visual stimulation. Glaucomatous mice exhibit prolonged N1 latency and diminished P1-N1 amplitude in VEP. These observations are in agreement with that seen in human patients.^{37,38} Baseline VEP is recorded at 1-2 days before microbead injection.

In all experimental groups, VEP and SD-OCT is assessed at 4, 8, and 12 weeks after PBS or microbead injection for continuing evaluation of visual function and retinal morphology changes. pSTR and OKR are analyzed through 12 weeks post PBS or microbead injection. Mice are sacrificed at 12 weeks after PBS or microbead injection, following the last VEP, OKR, pSTR, and SD-OCT assessments. Retinal and optic nerve sections are prepared as described previously.²⁹ Retinal sections are immunolabeled with antibody against an RGC specific marker, such as Tuj 1 or Brn3b, and counterstained by nuclear marker DAPI to reveal host RGC morphology and retinal laminar structure. Tumor formation are monitored by SD-OCT in live animals as well as examined in retinal sections. Axon counts are carried out in optic nerve sections as described²⁹ to measure axon degeneration/protection after RGCP transplantation.

Grafted cell survival at these later time points is evaluated in mice with and without IOP elevation (PBS-injection vs. microbead-injection) and also compared between groups of mice with or without receiving immune suppression.

In addition, a clinically applicable non-invasive imaging technology, spectral domain optical coherent tomography (SD-OCT), is used to monitor retinal morphology that includes measurements of potential tumor formation as well as ganglion cell layer (GCL) and nerve fiber layer (NFL) thickness. Others have reported the successful application of SD-OCT for quantification of thinning of the ganglion cell complex (GCC) layer (which includes the GCL and NFL) in live glaucomatous mice;³⁰ a finding that is in agreement with that seen in patients with glaucoma.

### Statistical analysis

Data are presented as mean ± standard deviation and analyzed with GraphPad Prism 5.01 for Windows (GraphPad Software Inc). Paired ANOVA and Mann-Whiney are used for statistical analysis. Differences are to be considered significant at p < 0.05.

### Results

As demonstrated in the earlier example, a novel procedure has been developed in accordance with this disclosure that allows robust differentiation of human PSCs to generate a highly homogenous (>95%) population of RGCPs. Moreover, when such RGCPs were transplanted into glaucomatous mice, significantly improved RGC survival (FIG. 13A) and function (FIGs. 14A-D), as measured by immunohistochemistry, pSTR and OKR, was observed. These data indicate that the transplanted RGCPs are able to preserve vision in glaucoma.

### Example 5: Characterization of RGC-like cells post RGCP transplantation

Example 5 investigates (1) the percentage of RGCPs differentiating into RGC-like cells after transplantation into glaucomatous mouse retina, (2) the morphological and molecular properties of human ESC and iPSC-derived RGC-like cells using immunohistochemistry and RNA sequencing (RNA-seq), and (3) analyzes the gene profile of these cells.

### RGCP transplantation into glaucoma mice and quantification for percentage of RGCP differentiation into RGC-like cells

RGCPs are generated from GFP+ hESCs or iPSCs as described in Examples 1 and 4. Adult C57BL/6J mice receive anterior chamber injections of microbeads to induce ocular hypertension in this glaucoma model. ²⁹ At 7 days after microbead injection, GFP-hESC- or HA1-iPSC-derived RGCPs (100,000 cells/µl) are injected intravitreally into the glaucomatous mice, with or without daily treatment of cyclosporine-A. While preliminary analysis has not detected any abnormalities in RGCP survival and differentiation in the absence of cyclosporine-A, comparison of cell phenotypes and transcriptomes provides additional information regarding the effect of an immune suppressor on engrafted RGCPs. To quantitatively assess RGCP differentiation, retinal flat-mounts are collected at 3 weeks post transplantation and immunolabeled for RGC specific markers, e.g., Brn3b and Tuj1. Engrafted RGCPs are identified by GFP expression or human specific mitochondrial antibody. Engrafted cells (GFP+) and RGC-like cells (GFP+/Brn3b+) are counted, respectively, and percentage of engrafted RGCPs becoming RGC-like cells (GFP+/Brn3b+) are calculated. Immunodetection for other retinal cell markers (e.g., amacrine cells, bipolar cells, photoreceptors, and Müller cells) are also carried out as described previously9^{28,39} to determine if engrafted RGCPs develop into other retinal cell types. Immunolabeling of pre- and post-synaptic markers, such as synaptotagmin, PSD-95, GluR2/3, is also performed, and co-localization of pre- (e.g., synaptotagmin) and post- (e.g., PSC95) synaptic markers suggests formation of functional synapses.^{40,41}

### Verification for genome-wide signs of RGC differentiation

Engrafted RGCPs are isolated from the mouse retinas at 3 weeks post transplantation (to allow cell differentiation), based on their GFP expression with FACS. Total RNAs is extracted from isolated GFP+ RGCPs, as well as from cultured EFPs (verified by Pax6+, Rx1+, Nestin+ and Sox2+ expression) and RGCPs (Neurofilement+/Brn3a+ /Math5-). ∼40,000 engrafted cells, which could be easily isolated from 4 injected retinas, are sufficient to provide the required amount of RNAs for this analysis. Briefly, libraries for RNA-Seq are generated using Illumina Truseq kit v2 and sequenced with Illumina HiSeq. The sequences are aligned to the human transcriptome to produce an aligned BAM file, and known genes and transcripts are quantified using the Cufflinks algorithm. Differential expression of genes and known transcripts are determined with gene annotation. Gene Set Enrichment Analysis with GSEA algorithm and Ingenuity Pathway Analysis are performed. Interesting candidate transcripts are verified by qPCR. Data obtained from engrafted RGCPs, and EFPs and RGCPs directly taken from ESC- and iPSC-derived cultures are compared. The expression of genes corresponding to RGC lineage identity (e.g., POU, Islet1, Islet2, Thy1.1)⁴² is monitored as cells differentiate from EFPs to RGCPs, and also in RGC-like cells produced *in vivo* by the differentiation of the transplanted RGCPs. Expression of other retinal cell genes, such as photoreceptor specific markers, is also monitored in RGCPs and RGC-like cells.

### Statistical analysis

Data are presented as mean ± standard deviation and analyzed with GraphPad Prism 5.01 for Windows (GraphPad Software Inc). Paired ANOVA and Mann-Whiney are be used for statistical analysis. Differences are be considered significant at p < 0.05.

### Example 6: Transplantation of RGCPs

Example 6 investigates if transplantation of RGCPs reverses vision loss or restores function by differentiation of the transplanted cells into mature RGCs and long axon extension into the optic nerve that connects to the brain.

### RGCP transplantation into optic nerve crush injured mice

RGCPs were generated from GFP+ hESCs or iPSCs as described in Examples 1 and 4. GFP+ hESC-RGCPs were used since it was easier to follow the fate of hESC-RGCPs after transplantation due to their GFP expression. Adult C57BL/6J mice received optic nerve crush injury that, as previously described, lead to complete damage of all optic nerve axons.²² GFP+ hESC-RGCPs (100,000 cells/µl) or vehicle control solution was injected intravitreally in these mice at 7 days post injury. Mice were sacrificed at 8 weeks post-injury.

Retinal flat-mounts and longitudinal optic nerve sections were prepared for quantification of RGC survival, differentiation, and nerve growth as follows: (1) to analyze RGC survival and differentiation, retinal flat-mounts were immunolabeled with Tuj 1 to identify endogenous (host) and new (GFP+) RGCs. Numbers of surviving host RGCs (Tuj1+ only) and numbers of newly generated RGCs (GFP+/Tuj 1+) were recorded; percentage of transplanted (GFP+) cells expressing RGC marker Tuj 1 were calculated (by dividing GFP+/Tuj 1+ cells by total GFP+ cells counted). Immunodetection for other retinal cell markers was also carried out to determine if engrafted RGCPs develop into additional cell types; and (2) to evaluate axon survival and regrowth, longitudinal optic nerve sections were stained with primary antibody against neurofilament protein to reveal endogenous axons, whereas new axons were identified by GFP labeling.

It is expected that all of the host axons are damaged and degenerated beyond the crush site by 4 weeks after injury and that only regenerating axons are to be found posterior to the crush site. To this end, numbers of and distances traveled by new GFP+ axons in longitudinal nerve sections were quantified. To determine if new axons reached the central targets in the brain, mouse brain sections were collected; numbers of GFP+ axons found in the optic tract and central targets of the optic nerve, including the lateral geniculate nucleus and superior colliculus, were recorded. Moreover, immunolabeling for synaptic markers, such as synaptophysin and PSD95, were performed in brain sections to determine if GFP+ axons developed synapses and express presynaptic markers.

Other mice are sacrificed at 4, 8, and 12 weeks post-injury. Potential functional benefit of RGCP transplantation is assessed by recording VEP, pSTR, and OKR at 1-2 days before injury and before sacrifice. SD-OCT is also performed to monitor retinal morphology in live mice.

### Statistical analysis

Data are presented as mean ± standard deviation and analyzed with GraphPad Prism 5.01 for Windows (GraphPad Software Inc). Paired ANOVA and Mann-Whiney are be used for statistical analysis. Differences are be considered significant at p < 0.05.

### Results

At 3 weeks after optic nerve injury, GFP+ cells repopulated the host retina and extended long neurites. Nearly all of the transplanted cells (>95%) differentiated into Tuj1+ RGCs (FIGs. 16A-C). Using both confocal and light sheet microscopy, engrafted RGCPs were observed to integrate into the ganglion cell layer (FIGs. 16D-E) and develop functional synapses as demonstrated by co-localization of pre- and post-synaptic markers (Figs. 16F-J). These data support that hESC-derived RGCPs can differentiate into RGC-like cells, integrate into the host retina, develop functional synapses, and replace lost cells following transplantation into optic nerve injured mice.

### Conclusion

To overcome the limitation in providing pure, renewable populations of human retinal ganglion cell progenitors (RGCPs) for research and therapy, a defined method has been developed for robust differentiation of human pluripotent stem cells (PSCs) into retinal neurons. This method has been carried out using multiple hESC and iPSC lines (5 hESC lines from blastocysts or single blastomeres; 6 iPSC lines obtained by episomal vectors or mRNA reprogramming) to generate a highly homogenous (>95%) population of RGCPs. This method was able to synchronize the retinal differentiation process in all these lines, consistently leading to a stepwise differentiation of PSCs into eye field progenitors (EFPs, FIG. 2)¹⁶⁻¹⁸, early RGCPs (Math5+, FIGs. 3B and 4)¹⁹⁻²¹, and late RGCPs (Math5-/Thy1.1+ FIGs. 3B , 4 and 5) that led to the generation of mature RGCs *in vitro* and *in vivo* (FIGs. 13A, 14A-B, and 16A-J).

In a preliminary study to test the therapeutic potential of these cells, GFP+ hESC-RGCPs were injected into the vitreous of mice with induced glaucoma. Significantly improved RGC survival was observed (FIG. 13A, lower panel), accompanied by increased amplitude of positive scotopic threshold response (pSTR), a measure of RGC function (FIG. 14A-C), in glaucomatous mice that received RGCP transplantation.

To further establish the therapeutic potential of RGCPs in severe optic neuropathy, the ability of such cells to replace lost RGCs and form new axons in an optic nerve crush injury model was analyzed. The results revealed that all of the transplanted cells differentiated into Tuj1+ RGCs and showed robust migration, integration, and formation of new neurites 3 weeks after cell injection (FIG. 16A-J).

These combined results indicate that human PSC-RGCPs can be used for glaucoma therapy and may also be used for the treatment of other forms of optic neuropathy.

**TABLE 1.**

| **Composition of B27 Medium Supplement** | | | |
|---|---|---|---|
| Biotin | | | |
| L-carnitine | | | |
| Corticosterone | | | |
| Ethanolamine | | | |
| D(+)-galactose | | | |
| Glutathione (reduced) | | | |
| Linoleic acid | | | |
| Linolenic acid | | | |
| Progesterone | | | |
| Putrescine | | | |
| Retinyl acetate | | | |
| Selenium | | | |
| T3 (triodo-1-thyronine) | | | |
| DL-alpha-tocopherol (vitamin E) | | | |
| DL-alpha-tocopherol acetate | | | |
| Proteins: | | | |
| Albumin, bovine | | | |
| Catalase | | | |
| Insulin | | | |
| Superoxide dismutase | | | |
| Superoxide dismutase | | | |

| **Composition of DMEM/F12 medium** | | | |
|---|---|---|---|
| **Components** | **Molecular Weight** | **Concentration (mg/L)** | **mM** |
| **Amino Acids** | | | |
| Glycine | 75 | 18.75 | 0.25 |
| L-Alanine | 89 | 4.45 | 0.05 |
| L-Arginine hydrochloride | 211 | 147.5 | 0.699052 |
| L-Asparagine-H2O | 150 | 7.5 | 0.05 |
| L-Aspartic acid | 133 | 6.65 | 0.05 |
| L-Cysteine hydrochloride-H2O | 176 | 17.56 | 0.099773 |
| L-Cystine 2HCl | 313 | 31.29 | 0.099968 |
| L-Glutamic Acid | 147 | 7.35 | 0.05 |
| L-Glutamine | 146 | 365 | 2.5 |
| L-Histidine hydrochloride-H2O | 210 | 31.48 | 0.149905 |
| L-Isoleucine | 131 | 54.47 | 0.415802 |
| L-Leucine | 131 | 59.05 | 0.450763 |
| L-Lysine hydrochloride | 183 | 91.25 | 0.498634 |
| L-Methionine | 149 | 17.24 | 0.115705 |
| L-Phenylalanine | 165 | 35.48 | 0.21503 |
| L-Proline | 115 | 17.25 | 0.15 |
| L-Serine | 105 | 26.25 | 0.25 |
| L-Threonine | 119 | 53.45 | 0.44916 |
| L-Tryptophan | 204 | 9.02 | 0.044216 |
| L-Tyrosine disodium salt dihydrate | 261 | 55.79 | 0.213755 |
| L-Valine | 117 | 52.85 | 0.451709 |

| **Vitamins** | | | |
|---|---|---|---|
| Biotin | 244 | 0.0035 | 1.43E-05 |
| Choline chloride | 140 | 8.98 | 0.064143 |
| D-Calcium pantothenate | 477 | 2.24 | 0.004696 |
| Folic Acid | 441 | 2.65 | 0.006009 |
| Niacinamide | 122 | 2.02 | 0.016557 |
| Pyridoxine hydrochloride | 206 | 2 | 0.009709 |
| Riboflavin | 376 | 0.219 | 5.82E-04 |
| Thiamine hydrochloride | 337 | 2.17 | 0.006439 |
| Vitamin B12 | 1355 | 0.68 | 5.02E-04 |
| i-Inositol | 180 | 12.6 | 0.07 |

| **Inorganic Salts** | | | |
|---|---|---|---|
| Calcium Chloride (CaCl2) (anhyd.) | 111 | 116.6 | 1.05045 |
| Cupric sulfate (CuSO4-5H2O) | 250 | 0.0013 | 5.20E-06 |
| Ferric Nitrate (Fe(NO3)3"9H2O) | 404 | 0.05 | 1.24E-04 |
| Ferrous Sulfate (FeSO4 7H2O) | 278 | 0.417 | 0.0015 |
| Magnesium Chloride (anhydrous) | 95 | 28.64 | 0.301474 |
| Magnesium Sulfate (MgSO4) (anhyd.) | 120 | 48.84 | 0.407 |
| Potassium Chloride (KCl) | 75 | 311.8 | 4.157333 |
| Sodium Bicarbonate (NaHCO3) | 84 | 1200 | 14.28571 |
| Sodium Chloride (NaCl) | 58 | 6995.5 | 120.6121 |
| Sodium Phosphate dibasic (Na2HPO4) anhydrous | 142 | 71.02 | 0.500141 |
| Sodium Phosphate monobasic (NaH2PO4-H2O) | 138 | 62.5 | 0.452899 |
| Zinc sulfate (ZnSO4-7H2O) | 288 | 0.432 | 0.0015 |

| **Other Components** | | | |
|---|---|---|---|
| D-Glucose (Dextrose) | 180 | 3151 | 17.50556 |
| HEPES | 238 | 3574.5 | 15.01891 |
| Hypoxanthine Na | 159 | 2.39 | 0.015031 |
| Linoleic Acid | 280 | 0.042 | 1.50E-04 |
| Lipoic Acid | 206 | 0.105 | 5.10E-04 |
| Phenol Red | 376.4 | 8.1 | 0.02152 |
| Putrescine 2HCl | 161 | 0.081 | 5.03E-04 |
| Sodium Pyruvate | 110 | 55 | 0.5 |
| Thymidine | 242 | 0.365 | 0.001508 |

| **Composition of Neurobasal™ Medium (Life Technologies)** | | | |
|---|---|---|---|
| **Components** | **Molecular Weight** | **Concentration (mg/L)** | **mM** |
| **Amino Acids** | | | |
| Glycine | 75 | 30 | 0.4 |
| L-Alanine | 89 | 2 | 0.022472 |
| L-Arginine hydrochloride | 211 | 84 | 0.398104 |
| L-Asparagine-H2O | 150 | 0.83 | 0.005533 |
| L-Cysteine | 121 | 31.5 | 0.260331 |
| L-Histidine hydrochloride-H2O | 210 | 42 | 0.2 |
| L-Isoleucine | 131 | 105 | 0.801527 |
| L-Leucine | 131 | 105 | 0.801527 |
| L-Lysine hydrochloride | 183 | 146 | 0.797814 |
| L-Methionine | 149 | 30 | 0.201342 |
| L-Phenylalanine | 165 | 66 | 0.4 |
| L-Proline | 115 | 7.76 | 0.067478 |
| L-Serine | 105 | 42 | 0.4 |
| L-Threonine | 119 | 95 | 0.798319 |
| L-Tryptophan | 204 | 16 | 0.078431 |
| L-Tyrosine | 181 | 72 | 0.39779 |
| L-Valine | 117 | 94 | 0.803419 |

| **Vitamins** | | | |
|---|---|---|---|
| Choline chloride | 140 | 4 | 0.028571 |
| D-Calcium pantothenate | 477 | 4 | 0.008386 |
| Folic Acid | 441 | 4 | 0.00907 |
| Niacinamide | 122 | 4 | 0.032787 |
| Pyridoxine hydrochloride | 204 | 4 | 0.019608 |
| Riboflavin | 376 | 0.4 | 0.001064 |
| Thiamine hydrochloride | 337 | 4 | 0.011869 |
| Vitamin B12 | 1355 | 0.0068 | 5.02E-06 |
| i-Inositol | 180 | 7.2 | 0.04 |

| Inorganic Salts | | | |
|---|---|---|---|
| Calcium Chloride (CaCl2) (anhyd.) | 111 | 200 | 1.801802 |
| Ferric Nitrate (Fe(NO3)3"9H2O) | 404 | 0.1 | 2.48E-04 |
| Magnesium Chloride (anhydrous) | 95 | 77.3 | 0.813684 |
| Potassium Chloride (KCl) | 75 | 400 | 5.333334 |
| Sodium Bicarbonate (NaHCO3) | 84 | 2200 | 26.19048 |
| Sodium Chloride (NaCl) | 58 | 3000 | 51.72414 |
| Sodium Phosphate monobasic (NaH2PO4-H2O) | 138 | 125 | 0.905797 |
| Zinc sulfate (ZnSO4-7H2O) | 288 | 0.194 | 6.74E-04 |

| **Other Components** | | | |
|---|---|---|---|
| D-Glucose (Dextrose) | 180 | 4500 | 25 |
| HEPES | 238 | 2600 | 10.92437 |
| Phenol Red | 376.4 | 8.1 | 0.02152 |
| Sodium Pyruvate | 110 | 25 | 0.227273 |

| **Composition of N2 Supplement** | | | |
|---|---|---|---|
| **Components** | **Molecular Weight** | **Concentration (mg/L)** | **mM** |
| **Proteins** | | | |
| Human Transferrin (Holo) | 10000 | 10000 | 1 |
| Insulin Recombinant Full Chain | 5807.7 | 500 | 0.086093 |

| **Other Components** | | | |
|---|---|---|---|
| Progesterone | 314.47 | 0.63 | 0.002003 |
| Putrescine | 161 | 1611 | 10.00621 |
| Selenite | 173 | 0.52 | 0.003006 |

### References:

[1] Quigley HA, Broman AT: The number of people with glaucoma worldwide in 2010 and 2020. The British journal of ophthalmology 2006, 90:262-7.
[2] Quigley HA: Glaucoma. Lancet 2011, 377:1367-77.
[3] Lund RD, Wang S, Klimanskaya I, Holmes T, Ramos-Kelsey R, Lu B, Girman S, Bischoff N, Sauve Y, Lanza R: Human embryonic stem cell-derived cells rescue visual function in dystrophic RCS rats. Cloning and stem cells 2006, 8:189-99.
[4] Lu B, Malcuit C, Wang S, Girman S, Francis P, Lemieux L, Lanza R, Lund R: Long-term safety and function of RPE from human embryonic stem cells in preclinical models of macular degeneration. Stem cells 2009, 27:2126-35.
[5] Schwartz SD, Regillo CD, Lam BL, Eliott D, Rosenfeld PJ, Gregori NZ, Hubschman JP, Davis JL, Heilwell G, Spirn M, Maguire J, Gay R, Bateman J, Ostrick RM, Morris D, Vincent M, Anglade E, Del Priore LV, Lanza R: Human embryonic stem cell-derived retinal pigment epithelium in patients with age-related macular degeneration and Stargardt's macular dystrophy: follow-up of two open-label phase 1/2 studies. Lancet 2015, 385:509-16.
[6] Parameswaran S, Balasubramanian S, Babai N, Qiu F, Eudy JD, Thoreson WB, Ahmad I: Induced pluripotent stem cells generate both retinal ganglion cells and photoreceptors: therapeutic implications in degenerative changes in glaucoma and age-related macular degeneration. Stem cells 2010, 28:695-703.
[7] Chen M, Chen Q, Sun X, Shen W, Liu B, Zhong X, Leng Y, Li C, Zhang W, Chai F, Huang B, Gao Q, Xiang AP, Zhuo Y, Ge J: Generation of retinal ganglion-like cells from reprogrammed mouse fibroblasts: IOVS 2010; 51(11): 5970-5978. Annals of neurosciences 2011, 18:64-5.
[8] Satarian L, Javan M, Kiani S, Hajikaram M, Mirnajafi-Zadeh J, Baharvand H: Engrafted human induced pluripotent stem cell-derived anterior specified neural progenitors protect the rat crushed optic nerve. PloS one 2013, 8:e71855.
[9] Hambright D, Park KY, Brooks M, McKay R, Swaroop A, Nasonkin IO: Long-term survival and differentiation of retinal neurons derived from human embryonic stem cell lines in un-immunosuppressed mouse retina. Molecular vision 2012, 18:920-36.
[10] Jagatha B, Divya MS, Sanalkumar R, Indulekha CL, Vidyanand S, Divya TS, Das AV, James J: In vitro differentiation of retinal ganglion-like cells from embryonic stem cell derived neural progenitors. Biochemical and biophysical research communications 2009, 380:230-5.
[11] Johnson TV, Bull ND, Hunt DP, Marina N, Tomarev SI, Martin KR: Neuroprotective effects of intravitreal mesenchymal stem cell transplantation in experimental glaucoma. Investigative ophthalmology & visual science 2010, 51:2051-9.
[12] Hu Y, Tan HB, Wang XM, Rong H, Cui HP, Cui H: Bone marrow mesenchymal stem cells protect against retinal ganglion cell loss in aged rats with glaucoma. Clinical interventions in aging 2013, 8:1467-70.
[13] Kassis I, Vaknin-Dembinsky A, Karussis D: Bone marrow mesenchymal stem cells: agents of immunomodulation and neuroprotection. Current stem cell research & therapy 2011, 6:63-8.
[14] Dreixler JC, Poston JN, Balyasnikova I, Shaikh AR, Tupper KY, Conway S, Boddapati V, Marcet MM, Lesniak MS, Roth S: Delayed administration of bone marrow mesenchymal stem cell conditioned medium significantly improves outcome after retinal ischemia in rats. Investigative ophthalmology & visual science 2014, 55:3785-96.
[15] Bull ND, Irvine KA, Franklin RJ, Martin KR: Transplanted oligodendrocyte precursor cells reduce neurodegeneration in a model of glaucoma. Investigative ophthalmology & visual science 2009, 50:4244-53.
[16] Wawersik S, Maas RL: Vertebrate eye development as modeled in Drosophila. Human molecular genetics 2000, 9:917-25.
[17] Zaghloul NA, Yan B, Moody SA: Step-wise specification of retinal stem cells during normal embryogenesis. Biology of the cell / under the auspices of the European Cell Biology Organization 2005, 97:321-37.
[18] Zuber ME, Gestri G, Viczian AS, Barsacchi G, Harris WA: Specification of the vertebrate eye by a network of eye field transcription factors. Development 2003, 130:5155-67.
[19] Brown NL, Kanekar S, Vetter ML, Tucker PK, Gemza DL, Glaser T: Math5 encodes a murine basic helix-loop-helix transcription factor expressed during early stages of retinal neurogenesis. Development 1998, 125:4821-33.
[20] Brown NL, Patel S, Brzezinski J, Glaser T: Math5 is required for retinal ganglion cell and optic nerve formation. Development 2001, 128:2497-508.
[21] Wang SW, Kim BS, Ding K, Wang H, Sun D, Johnson RL, Klein WH, Gan L: Requirement for math5 in the development of retinal ganglion cells. Genes & development 2001, 15:24-9.
[22] Koprivica V, Cho KS, Park JB, Yiu G, Atwal J, Gore B, Kim JA, Lin E, Tessier-Lavigne M, Chen DF, He Z: EGFR activation mediates inhibition of axon regeneration by myelin and chondroitin sulfate proteoglycans. Science 2005, 310:106-10.
[23] Hu Y, Park KK, Yang L, Wei X, Yang Q, Cho KS, Thielen P, Lee AH, Cartoni R, Glimcher LH, Chen DF, He Z: Differential effects of unfolded protein response pathways on axon injury-induced death of retinal ganglion cells. Neuron 2012, 73:445-52.
[24] Zhang XM, Li Liu DT, Chiang SW, Choy KW, Pang CP, Lam DS, Yam GH: Immunopanning purification and long-term culture of human retinal ganglion cells. Mol Vis 2010, 16:2867-72.
[25] Morishita S, Oku H, Horie T, Tonari M, Kida T, Okubo A, Sugiyama T, Takai S, Hara H, Ikeda T: Systemic simvastatin rescues retinal ganglion cells from optic nerve injury possibly through suppression of astroglial NF-kappaB activation. PLoS One 2014, 9:e84387.
[26] Cho KS, Yang L, Lu B, Feng Ma H, Huang X, Pekny M, Chen DF: Re-establishing the regenerative potential of central nervous system axons in postnatal mice. Journal of cell science 2005, 118:863-72.
[27] Huang X, Wu DY, Chen G, Manji H, Chen DF: Support of retinal ganglion cell survival and axon regeneration by lithium through a Bcl-2-dependent mechanism. Investigative ophthalmology & visual science 2003, 44:347-54.
[28] Kinouchi R, Takeda M, Yang L, Wilhelmsson U, Lundkvist A, Pekny M, Chen DF: Robust neural integration from retinal transplants in mice deficient in GFAP and vimentin. Nature neuroscience 2003, 6:863-8.
[29] Chen H, Wei X, Cho KS, Chen G, Sappington R, Calkins DJ, Chen DF: Optic neuropathy due to microbead-induced elevated intraocular pressure in the mouse. Invest Ophthalmol Vis Sci 2011, 52:36-44.
[30] Yang Q, Cho KS, Chen H, Yu D, Wang WH, Luo G, Pang IH, Guo W, Chen DF: Microbead-induced ocular hypertensive mouse model for screening and testing of aqueous production suppressants for glaucoma. Investigative ophthalmology & visual science 2012, 53:3733-41.
[31] Feng Q, Shabrani N, Thon JN, Huo H, Thiel A, Machlus KR, Kim K, Brooks J, Li F, Luo C, Kimbrel EA, Wang J, Kim KS, Italiano J, Cho J, Lu SJ, Lanza R: Scalable generation of universal platelets from human induced pluripotent stem cells. Stem cell reports 2014, 3:817-31.
[32] Chung Y, Klimanskaya I, Becker S, Li T, Maserati M, Lu SJ, Zdravkovic T, Ilic D, Genbacev O, Fisher S, Krtolica A, Lanza R: Human embryonic stem cell lines generated without embryo destruction. Cell stem cell 2008, 2:113-7.
[33] Lu SJ, Feng Q, Park JS, Vida L, Lee BS, Strausbauch M, Wettstein PJ, Honig GR, Lanza R: Biologic properties and enucleation of red blood cells from human embryonic stem cells. Blood 2008, 112:4475-84.
[34] Lu SJ, Li F, Yin H, Feng Q, Kimbrel EA, Hahm E, Thon JN, Wang W, Italiano JE, Cho J, Lanza R: Platelets generated from human embryonic stem cells are functional in vitro and in the microcirculation of living mice. Cell research 2011, 21:530-45.
[35] Burroughs SL, Kaja S, Koulen P: Quantification of deficits in spatial visual function of mouse models for glaucoma. Investigative ophthalmology & visual science 2011, 52:3654-9.
[36] Domenici L, Origlia N, Falsini B, Cerri E, Barloscio D, Fabiani C, Sanso M, Giovannini L: Rescue of retinal function by BDNF in a mouse model of glaucoma. PloS one 2014, 9:e115579.
[37] Atkin A, Bodis-Wollner I, Podos SM, Wolkstein M, Mylin L, Nitzberg S: Flicker threshold and pattern VEP latency in ocular hypertension and glaucoma. Investigative ophthalmology & visual science 1983, 24:1524-8.
[38] Greenstein VC, Seliger S, Zemon V, Ritch R: Visual evoked potential assessment of the effects of glaucoma on visual subsystems. Vision research 1998, 38:1901-11.
[39] Takeda M, Takamiya A, Jiao JW, Cho KS, Trevino SG, Matsuda T, Chen DF: alpha-Aminoadipate induces progenitor cell properties of Muller glia in adult mice. Investigative ophthalmology & visual science 2008, 49:1142-50.
[40] Miller RF, Fagerson MH, Staff NP, Wolfe R, Doerr T, Gottesman J, Sikora MA, Schuneman R: Structure and functional connections of presynaptic terminals in the vertebrate retina revealed by activity-dependent dyes and confocal microscopy. The Journal of comparative neurology 2001, 437:129-55.
[41] Ullian EM, Sapperstein SK, Christopherson KS, Barres BA: Control of synapse number by glia. Science 2001, 291:657-61.
[42] Mu X, Klein WH: A gene regulatory hierarchy for retinal ganglion cell specification and differentiation. Seminars in cell & developmental biology 2004, 15:115-23.

## Claims

1. A method of producing a plurality of cells, comprising
culturing eye field progenitor cells, as cell clusters and under low adherence or non-adherent conditions, in a cell media for a period of time sufficient for the cell clusters to form a plurality of cells,
wherein the eye field progenitor cells are capable of differentiating into retinal ganglion progenitor cells in the cell media, and wherein the cell media comprises forskolin, BDNF, and/or CNTF,
wherein the plurality of cells express:
one or more of Math5, Brn3a, Brn3b, Isl1, Neurofilament, and Thy1,
wherein the eye field progenitor cells are characterized as Pax6(+), Rx1(+), Oct4(-) and Nanog(-), and preferably are also characterized as Six3(+), Six6(+), Lhx2(+), Tbx3(+), Sox2(+), Otx2(+) and Nestin(+), as determined by immunostaining and/or flow cytometry.

2. The method of claim 1, further comprising culturing pluripotent stem cells under feeder-free conditions to produce the eye field progenitor cells.

3. The method of claim 1 or 2, further comprising producing a plurality of cells by culturing the cells in a retinal ganglion cell (RGC) differentiation medium.

4. The method of claim 3, wherein the plurality of cells express:
one or more of Brn3a, Neurofilament, Tuj1, and Thy1.

5. A composition comprising a plurality of cells produced according to the method of any one of claims 1-4.

6. A composition of cells, comprising
a plurality of cells at least 60% of which express
(i) one or more of Brn3a, Neurofilament, and Thyl, or
(ii) one or more of Math5, Brn3a, Brn3b, and Isl1,
as determined immunocytochemically,
and wherein the plurality of cells are formed as neurospheres.

7. The composition of claim 6(ii),
(a) further comprising a medium suitable for maintaining the viability of the cells, or
(b) wherein the composition is substantially free of pluripotent stem cells, mature photoreceptors, and/or amacrine cells.

8. The composition of claim 6, wherein the composition is a pharmaceutical preparation that is suitable for use in a mammalian patient, and further comprises a pharmaceutically acceptable carrier for maintaining the viability of the cells for transplantation into a mammalian patient.

9. The composition of claim 6, wherein the composition is a cryogenic cell preparation, and optionally wherein at least 80% of the cell number initially frozen are recovered after thawing.

10. The composition of any one of claims 6-9, wherein the plurality of cells is derived from a pluripotent stem cell *in vitro.*

11. The composition of claim 6(ii), wherein at least 50% of the cells in plurality express
(1) Math5 and Brn3a,
(2) Math5, Brn3a and Brn3b, or
(3) Math5, Brn3a, Brn3b and Isl1,
as determined immunocytochemically.

12. The composition of claim 6(i), wherein at least 50% of the cells in plurality express
(1) Brn3a and Neurofilament,
(2) Brn3a and Tuj 1,
(3) Brn3a, Tuj1 and Neurofilament, or
(4) Brn3a, Tuj1 and Thy1
as determined immunocytochemically.

13. The composition of any one of claims 6-12, wherein at least 75%, 85%, 95% or 99% of the cells in plurality express
(i) one or more of Brn3a, Neurofilament, Tuj1, and Thyl, or
(ii) one or more of Math5, Brn3a, Brn3b, Isl1, Neurofilament, and Thy1,
as determined immunocytochemically.

14. The composition of any one of claims 5-13, for use in a method of treatment of a mammalian patient in need thereof, comprising administering the composition to said patient, or administering the composition after thawing to said patient.

## Patentansprüche

1. Verfahren zur Herstellung einer Mehrzahl von Zellen, umfassend
Kultivieren von Augenfeld-Vorläuferzellen als Zellcluster und unter Bedingungen mit geringer Adhärenz oder ohne Adhärenz in einem Zellmedium für eine Zeitspanne, die ausreicht, damit die Zellcluster eine Mehrzahl von Zellen bilden,
wobei die Augenfeld-Vorläuferzellen in der Lage sind, sich in dem Zellmedium in Retinalganglien-Vorläuferzellen zu differenzieren, und wobei das Zellmedium Forskolin, BDNF und/oder CNTF umfasst,
wobei die Mehrzahl von Zellen exprimiert:
eines oder mehrere von Math5, Brn3a, Brn3b, Isl1, Neurofilament, und Thy1,
wobei die Augenfeld-Vorläuferzellen als Pax6(+), Rxl(+), Oct4(-) und Nanog(-) charakterisiert sind und vorzugsweise auch als Six3(+), Six6(+), Lhx2(+), Tbx3(+), Sox2(+), Otx2(+) und Nestin(+) charakterisiert sind, wie durch Immunfärbung und/oder Durchflusszytometrie bestimmt.

2. Verfahren nach Anspruch 1, ferner umfassend das Kultivieren von pluripotenten Stammzellen unter feederfreien Bedingungen, um die Augenfeld-Vorläuferzellen zu erzeugen.

3. Verfahren nach Anspruch 1 oder 2, ferner umfassend das Herstellen einer Mehrzahl von Zellen durch Kultivieren der Zellen in einem Differenzierungsmedium für Retinalganglienzellen (RGC).

4. Verfahren nach Anspruch 3, bei dem die Mehrzahl der Zellen eines oder mehrere von Brn3a, Neurofilament, Tuj1 und Thy1 exprimiert.

5. Zusammensetzung, umfassend eine Mehrzahl von nach dem Verfahren nach einem der Ansprüche 1-4 hergestellten Zellen.

6. Zellenzusammensetzung, umfassend
eine Mehrzahl von Zellen, von denen gemäß immunzytochemischer Bestimmung mindestens 60 %
(i) eines oder mehrere von Brn3a, Neurofilament und Thy1, oder
(ii) eines oder mehrere von Math5, Brn3a, Brn3b und Isl1 exprimieren,
und wobei die Mehrzahl von Zellen als Neurosphären ausgebildet sind.

7. Zusammensetzung nach Anspruch 6(ii),
(a) die ferner ein Medium umfasst, das geeignet ist, die Lebensfähigkeit der Zellen zu erhalten, oder
(b) bei der die Zusammensetzung im Wesentlichen frei von pluripotenten Stammzellen, reifen Photorezeptoren und/oder Amakrinzellen ist.

8. Zusammensetzung nach Anspruch 6, wobei die Zusammensetzung ein pharmazeutisches Präparat ist, das zur Verwendung in einem Säugetierpatienten geeignet ist, und ferner einen pharmazeutisch akzeptablen Träger zur Aufrechterhaltung der Lebensfähigkeit der Zellen für die Transplantation in einen Säugetierpatienten umfasst.

9. Zusammensetzung nach Anspruch 6, wobei die Zusammensetzung ein kryogenes Zellpräparat ist, und optional, wobei mindestens 80 % der ursprünglich eingefrorenen Zellzahl nach dem Auftauen wiedergewonnen werden.

10. Zusammensetzung nach einem der Ansprüche 6 bis 9, bei der die Mehrzahl von Zellen in vitro von einer pluripotenten Stammzelle abgeleitet ist.

11. Zusammensetzung nach Anspruch 6(ii), bei der gemäß immunzytochemischer Bestimmung mindestens 50% der Zellen der Mehrzahl (1) Math5 und Brn3a, (2) Math5, Brn3a und Brn3b, oder (3) Math5, Brn3a, Brn3b und Isl1 exprimieren.

12. Zusammensetzung nach Anspruch 6(i), bei der gemäß immunzytochemischer Bestimmung mindestens 50% der Zellen der Mehrzahl
(1) Brn3a und Neurofilament,
(2) Brn3a und Tuj 1,
(3) Brn3a, Tuj 1 und Neurofilament, oder
(4) Brn3a, Tuj1 und Thy1
exprimieren.

13. Zusammensetzung nach einem der Ansprüche 6-12, bei der gemäß immunzytochemischer Bestimmung mindestens 75 %, 85 %, 95 % oder 99 % der Zellen der Mehrzahl
(i) eines oder mehrere von Brn3a, Neurofilament, Tuj 1 und Thy1 oder
(ii) eines oder mehrere von Math5, Brn3a, Brn3b, Isl1, Neurofilament und Thy1
exprimieren.

14. Zusammensetzung nach einem der Ansprüche 5 bis 13 zur Verwendung in einem Verfahren zur Behandlung eines Säugetierpatienten, der diese benötigt, umfassend das Verabreichen der Zusammensetzung an den Patienten oder das Verabreichen der Zusammensetzung an den Patienten nach Auftauen.

## Revendications

1. Procédé de production d'une pluralité de cellules, comprenant les étapes consistant à
cultiver des cellules progénitrices de champ oculaire, sous forme de groupes de cellules et dans des conditions de faible adhérence ou de non-adhérence, dans un milieu cellulaire pendant une période de temps suffisante pour que les groupes de cellules forment une pluralité de cellules,
dans lequel les cellules progénitrices de champ oculaire sont capables de se différencier en cellules progénitrices de ganglion rétinien dans le milieu cellulaire, et dans lequel le milieu cellulaire comprend de la forskoline, du BDNF et/ou du CNTF,
dans lequel la pluralité de cellules exprime :
un ou plusieurs parmi Math5, Brn3a, Brn3b, Isl1, Neurofilament et Thy1,
dans lequel les cellules progénitrices de champ oculaire sont caractérisées comme Pax6(+), Rx1(+), Oct4(-) et Nanog(-), et sont de préférence également caractérisées comme Six3(+), Six6(+), Lhx2(+), Tbx3(+), Sox2(+), Otx2(+) et Nestin(+), comme déterminé par immunocoloration et/ou cytométrie en flux.

2. Procédé selon la revendication 1, comprenant en outre la culture de cellules souches pluripotentes dans des conditions sans cellules nourricières pour produire les cellules progénitrices de champ oculaire.

3. Procédé selon la revendication 1 ou 2, comprenant en outre la production d'une pluralité de cellules en cultivant les cellules dans un milieu de différenciation de cellules de ganglion rétinien (RGC).

4. Procédé selon la revendication 3, dans lequel la pluralité de cellules exprime :
un ou plusieurs parmi Brn3a, Neurofilament, Tuj1 et Thyl.

5. Composition comprenant une pluralité de cellules produites selon le procédé selon l'une quelconque des revendications 1 à 4.

6. Composition de cellules, comprenant
une pluralité de cellules dont au moins 60 % qui expriment
(i) un ou plusieurs parmi Brn3a, Neurofilament, et Thy1, ou
(ii) un ou plusieurs parmi Math5, Brn3a, Brn3b et Isl1, comme déterminé de manière immunocytochimique,
et dans laquelle la pluralité de cellules sont formées sous forme de neurosphères.

7. Composition selon la revendication 6(ii),
(a) comprenant en outre un milieu approprié pour maintenir la viabilité des cellules, ou
(b) dans laquelle la composition est sensiblement exempte de cellules souches pluripotentes, de photorécepteurs matures et/ou de cellules d'amacrine.

8. Composition selon la revendication 6, dans laquelle la composition est une préparation pharmaceutique qui est appropriée pour une utilisation dans un patient mammifère, et comprend en outre un support pharmaceutiquement acceptable pour maintenir la viabilité des cellules pour une transplantation chez un patient mammifère.

9. Composition selon la revendication 6, dans laquelle la composition est une préparation cellulaire cryogénique, et facultativement dans laquelle au moins 80 % du nombre de cellules initialement congelées sont récupérées après décongélation.

10. Composition selon l'une quelconque des revendications 6 à 9, dans laquelle la pluralité de cellules est dérivée d'une cellule souche pluripotente *in vitro.*

11. Composition selon la revendication 6(ii), dans laquelle au moins 50 % des cellules de la pluralité expriment
(1) Math5 et Brn3a,
(2) Math5, Brn3a et Brn3b, ou
(3) Math5, Brn3a, Brn3b et Isl1,
comme déterminé de manière immunocytochimique.

12. Composition selon la revendication 6(i), dans laquelle au moins 50 % des cellules de la pluralité expriment
(1) Brn3a et Neurofilament,
(2) Brn3a et Tuj1,
(3) Brn3a, Tuj1 et Neurofilament, ou
(4) Brn3a, Tuj1 et Thy1
comme déterminé de manière immunocytochimique.

13. Composition selon l'une quelconque des revendications 6 à 12, dans laquelle au moins 75 %, 85 %, 95 % ou 99 % des cellules de la pluralité expriment
(i) un ou plusieurs parmi Brn3a, Neurofilament, Tuj1, et Thy1, ou
(ii) un ou plusieurs parmi Math5, Brn3a, Brn3b, Isl1, Neurofilament et Thy1, comme déterminé de manière immunocytochimique.

14. Composition selon l'une quelconque des revendications 5 à 13, à utiliser dans un procédé de traitement d'un patient mammifère qui en a besoin, comprenant l'administration de la composition audit patient, ou l'administration de la composition après décongélation audit patient.
